# EUROPEAN PATENT SPECIFICATION

(11) **EP 4 158 565 B1**
(45) Date of publication and mention of the grant of the patent: **18.11.2026**
(21) Application number: 21728647.5
(22) Date of filing: 24.05.2021
(51) Int. Cl.: G06Q 10/0639, G06Q 50/00

(54) **HYGIENE MANAGEMENT SYSTEM, HYGIENE MANAGEMENT METHOD, AND HYGIENE MANAGEMENT PROGRAM**
HYGIENE MANAGEMENT SYSTEM, HYGIENE MANAGEMENT METHODE UND HYGIENE MANAGEMENT PROGRAMM
DISPOSITIF, PROCÉDÉ ET PROGRAMME DE GESTION DE L'HYGIÈNE

(30) Priority: 28.05.2020 JP 2020093360
(43) Date of publication of application: 05.04.2023
(73) Proprietor: Neogen Food Safety US HoldCo Corporation, Lansing MI 48912 (US)
(72) Inventor: SAITO, Shoko, Kawasaki-shi, Kanagawa 215-0006 (JP)
(74) Representative: Mathys & Squire
(86) International application number: PCT/IB2021/054490
(87) International publication number: WO 2021/240343

(56) References cited:
- WO-A1-2013/183635
- JP-A- 2008 059 507
- JP-A- 2010 067 239
- JP-A- 2017 201 992
- SG-A1- 193 641
- US-A1- 2014 046 722

## Description

### Technical Field

The present disclosure relates to a hygiene management system, a hygiene management method, and a hygiene management program.

### Background Art

Patent Document 1 describes a "hygiene management system capable of, for the purpose of managing data of a film-type medium of a general-purpose product which, by standards, is not provided with identification information for identifying the film-type medium from other film-type medium, issuing a medium label for attachment to the film-type medium, and the medium label includes a medium ID for identifying a medium from other medium and readable information including at least a portion of work information formed in a manner readable by a worker."

### Citation List : Patent Literature

Patent Document 1: JP 2014-026472 A
Patent Document 2: SG 193641 A1
Patent Document 3: JP 2008059507 A

### Summary of Invention

### Technical Problem

A hygiene management system is used by a plurality of facilities where testing for bacteria or the like is performed. In such facilities where testing is performed for a test target substance such as bacteria, hygiene management within the facilities is enabled by accumulating information in a database of a hygiene management system. However, information accumulated in a database is only viewed by a facility storing the information, and the information is not shared.

### Solution to Problem

A hygiene management system according to an aspect of the present disclosure is a hygiene management system for use by a plurality of facilities performing testing of a test target substance, the hygiene management system including:
a facility skill information reception unit configured to receive facility information and skill information of the facilities;
a facility skill information determining unit configured to determine a pass/fail for the facility information and the skill information; and
an authorization unit configured to authorize a facility of the facilities determined to have passed by the facility skill information determining unit to view hygiene management information of other facility of the facilities and/or be viewable by other facility of the facilities.

In the hygiene management system according to this aspect, the facility skill information reception unit receives the facility information and the skill information of the facilities and the facility skill information determining unit determines a pass/fail for the facility information and the skill information. In this hygiene management system, the authorization unit authorizes a facility of the facilities determined to have passed as a result of the pass/fail determination for the facility information and the skill information to view the hygiene management information of other facility of the facilities and/or to be viewable by other facility of the facilities. Accordingly, a facility determined to have passed as a result of a pass/fail determination for the facility information and the skill information is given the authorization, and thus, a facility that satisfies predetermined conditions can be allowed to share information with other facility. Thus, data can be shared while securing the reliability of database, and the usefulness of the hygiene management system can be improved.

The facility information may include ISO 17025 accreditation information.

The skill information may include a level of external accuracy management.

The hygiene management system according to another aspect may include a facility skill information update unit configured to request the facilities to update the facility information and the skill information in a certain amount of time after the authorization unit has given the authorization.

A hygiene management system according to another aspect of the present disclosure is a hygiene management system for use by a plurality of facilities performing testing of a test target substance, the hygiene management system including:
a storage unit configured to store hygiene management information of the test target substance of the plurality of facilities;
a keyword reception unit configured to receive, from the facilities, a search keyword for searching for the hygiene management information;
a hygiene management information extraction unit configured to extract, from the storage unit, hygiene management information matching the search keyword;
a hygiene management information transmission unit configured to transmit, to the facilities, the hygiene management information extracted by the hygiene management information extraction unit.

In the hygiene management system of this aspect, the storage unit stores the hygiene management information of the plurality of facilities, and the hygiene management information extraction unit extracts, from the storage unit, the hygiene management information matching the search keyword. Then, the extracted hygiene management information is transmitted to the facilities by the hygiene management information transmission unit. Accordingly, as with the hygiene management system described above, since the hygiene management information of other facility can be transmitted to a facility. the facility can view the hygiene management information of the other facility. As a result, the hygiene management information can be shared and the usefulness of the hygiene management system can be improved.

The hygiene management information extraction unit may extract information of the number of the test target substances obtained by the testing.

The hygiene management information extraction unit may extract image data of the test target substance obtained by the testing.

The hygiene management information extraction unit may extract test contents of the test target substance.

A hygiene management method according to an aspect of the present disclosure is a hygiene management method for use by a plurality of facilities performing testing of a test target substance, the method including the steps of: transmitting facility information and skill information of the facilities by the facilities; receiving pass/fail determination results of the facility information and the skill information; and viewing hygiene management information of other facility of the facilities by a facility of the facilities that has received pass determination results at the receiving step.

A hygiene management method according to another aspect of the present disclosure is a hygiene management method for use by a plurality of facilities performing testing of a test target substance, the method including the steps of: receiving, from the facilities, facility information and skill information of the facilities; determining a pass/fail of the facilities from the facility information and the skill information; transmitting the pass/fail to the facilities; and authorizing a facility of the facilities determined to have passed at the pass/fail determining step to view hygiene management information of other facility of the facilities and/or be viewable by other facility of the facilities.

A hygiene management program according to an aspect of the present disclosure is a hygiene management program for use by a plurality of facilities performing testing of a test target substance, the hygiene management program including the steps of: receiving, from the facilities, facility information and skill information of the facilities; determining a pass/fail of the facilities from the facility information and the skill information; transmitting the pass/fail to the facilities; and authorizing a facility of the facilities determined to have passed at the pass/fail determining step to view hygiene management information of other facility of the facilities and/or be viewable by other facility of the facilities.

In the hygiene management method and the hygiene management program of these aspects, the facility information and the skill information of the plurality of facilities are transmitted and received, and a pass/fail of the facilities is determined from the facility information and the skill information. Then, the facilities are notified of the determined pass/fail results, and a facility determined to have passed is authorized to view hygiene management information of other facility and/or be viewable by other facility. Accordingly, a facility determined to have passed can, for example, view hygiene management information of other facility in exchange for enabling other facility to view hygiene management information of the facility. Thus, the hygiene management information can be shared while securing the reliability of database, and the usefulness of the hygiene management system can be improved.

### Advantageous Effects of Invention

According to the present disclosure, the usefulness of a hygiene management system can be improved.

### Brief Description of Drawings

FIG. 1 is a view illustrating an example of a functional configuration of a hygiene management system.
FIG. 2 is a view illustrating an example of a hardware configuration of a server or a terminal used in a hygiene management system.
FIG. 3 is a view illustrating an example of a data configuration of a hygiene management system.
FIG. 4 is a view illustrating a detailed example of a master data of the data configuration of FIG. 3.
FIG. 5 is a view illustrating an example of contents of a testing data master of FIG. 4.
FIG. 6 is a flowchart illustrating an example of the steps of a hygiene management method.
FIG. 7 is a flowchart illustrating an example of the steps of a "member registration flow" of FIG. 6.
FIG. 8 is a sequence chart illustrating an example of the steps of "member qualification determination" of FIG. 7.
FIG. 9 is a view illustrating an example of a screen for "basic information registration" of FIG. 8.
FIG. 10 is a view illustrating an example of a screen for "facility information and skill information registration" of FIG. 8.
FIG. 11 is a view illustrating an example of a screen for "own data provision approval" of FIG. 7.
FIG. 12 is a flowchart illustrating an example of the steps of an "information update flow" of FIG. 6.
FIG. 13 is a flowchart illustrating a detailed example of the steps from "lot data generation" to "report generation" of FIG. 6.
FIG. 14 is a view illustrating an example of a "identification code output" of FIG. 13.
FIG. 15 is an explanatory view for information present in an identification code of FIG. 14.
FIG. 16 is a perspective view illustrating an exemplary plate used as a medium of FIG. 13.
FIG. 17 is a view illustrating an example of an aspect where an identification code is associated with the plate of FIG. 16.
FIG. 18 is a perspective view illustrating an exemplary reader that reads the plate of FIG. 16.
FIG. 19 is a view illustrating an example of a screen displaying a bacteria count (colony count) that is an example of the number of test target substances input via the reader of FIG. 18.
FIG. 20 is a view illustrating an example of a bacteria count data file including a bacteria count input via the reader of FIG. 18.
FIG. 21 is a view illustrating an example of a test results confirmation screen displayed at the "determination" step of FIG. 13.
FIG. 22 is a flowchart illustrating an example of the steps of a "data search flow" executed by a hygiene management system.
FIG. 23 is a view illustrating an example of a screen displayed at the "search keyword input" step of FIG. 22.
FIG. 24 is a view illustrating an example of a screen displayed at the "search results display" step of FIG. 22 (in a case where "test contents" is selected in FIG. 23).
FIG. 25 is a view illustrating an example of a screen displayed at the "search results display" step of FIG. 22 (in a case where an "image" is selected in FIG. 23).
FIG. 26 is a view illustrating an example of a screen displayed at the "search results display" step of FIG. 22 (in a case where a "bacteria count" is selected in FIG. 23).

### Description of Embodiments

A hygiene management system, a hygiene management method, and a hygiene management program according to the present disclosure will be described with reference to the drawings. In the description of the drawings, the same or equivalent elements are denoted by the same reference signs and redundant explanation will be omitted as appropriate. Additionally, in the drawings, a portion may be drawn in a simplified or exaggerated manner for ease of understanding, and a dimensional ratio and the like are not limited to those described in the drawings. Further, a layout of a screen used in the hygiene management system, the hygiene management method, and the hygiene management program is not limited to examples described below and can be changed as appropriate.

The hygiene management system, the hygiene management method, and the hygiene management program according to the present embodiment is, as an example, a system, a method, and a program for hygiene management of food. In the industry of hygiene management of food, for example, testing of a test target substance such as bacteria is performed within a facility, but information such as a method of testing and standards for testing varies depending on facilities. In the present circumstances, information of the method and the standards described above used in a hygiene management system, a hygiene management method, and a hygiene management program is not shared among a plurality of facilities. Thus, for example, when a testing method and standards for the own facility are determined, or when the validity of results obtained in the own facility is confirmed, information of other facility cannot be viewed and referred to. On the other hand, in simple information sharing among a plurality of facilities, there is a concern that due to mixed and accumulated information having low reliability, the reliability of a database may reduce.

In the hygiene management system, the hygiene management method, and the hygiene management program according to the present embodiment, for example, some of facilities can view hygiene management information of other facility. Thus, sharing of hygiene management information can be achieved and the reliability of database can be secured. That is, some of facilities can share hygiene management information with other facility. Thus, hygiene management information of other facility can be referred to while maintaining the reliability of database, and this contributes to improving the usefulness of the hygiene management system.

In the present disclosure, the "hygiene management information" refers to information relating to testing of a test target substance. For example, the "hygiene management information" includes at least any of testing standards, a test object, test contents, test results, and a testing method for a test target substance. Examples of the "test target substance" include various bacteria such as pathogenic bacteria and hygiene indicator bacteria, an allergen (protein and the like), and the like. The present embodiment will describe an example where the "test target substance" is hygiene indicator bacteria.

The "specimen" refers to an object to be tested and includes, for example, a measurement object where the number of bacteria (bacteria count), that are a kind of test target substance is measured. The "hygiene management" refers to managing a hygienic state of a specimen by measuring the number of test target substances such as pathogenic bacteria, allergens, or food hygiene bacteria, or by measuring the presence of a test target substance. For example, the hygiene management includes food hygiene management and environmental hygiene management.

In the present disclosure, the "facility" refers to a facility where testing for a test target substance is performed, and includes, for example, a company where testing is performed and a laboratory where testing is performed. One company may have a plurality of "facilities." The "facility information" refers to information of a facility where testing is performed, and includes, for example, equipment information relating to whether a facility has sufficient equipment to perform testing. The "skill information" refers to information relating to the skill of a tester that performs testing in a facility.

A hygiene management system 1 according to an embodiment is, for example, a computer system used to perform hygiene management for testing of a test target substance. The type of a test target substance is not limited in any way. The type and the configuration of an apparatus that functions as the hygiene management system 1 is not limited. For example, the hygiene management system 1 may include a single computer. The hygiene management system 1 may be, for example, a portable terminal such as a tablet terminal, a high-performance mobile phone (smartphone), and a laptop personal computer, or may be a desktop personal computer. Alternatively, the hygiene management system 1 may be a distributed processing system including a plurality of computers, or may be a client-server system or a cloud system. By collecting information from multiple facilities and making hygiene information available across different facilities, the hygiene management system 1 may enable facility management to optimize resource consumption and/or improve hygiene maintenance precision.

The hygiene management program according to the present embodiment includes, for example, a main module, a data acquisition module, an image analysis module, a determination module, and an output module. The main module is a module that comprehensively manages the functions of the hygiene management system 1. The data acquisition module, the image analysis module, the determination module, and the output module are executed, and thus respective functional components of the hygiene management system 1 function. The hygiene management program may be provided, for example, by being recorded in a non-rewritable manner in a physical recording medium such as a CD-ROM, a DVD-ROM, or a semiconductor memory. Alternatively, the hygiene management program may be provided via a communication network as a data signal superimposed on a carrier wave.

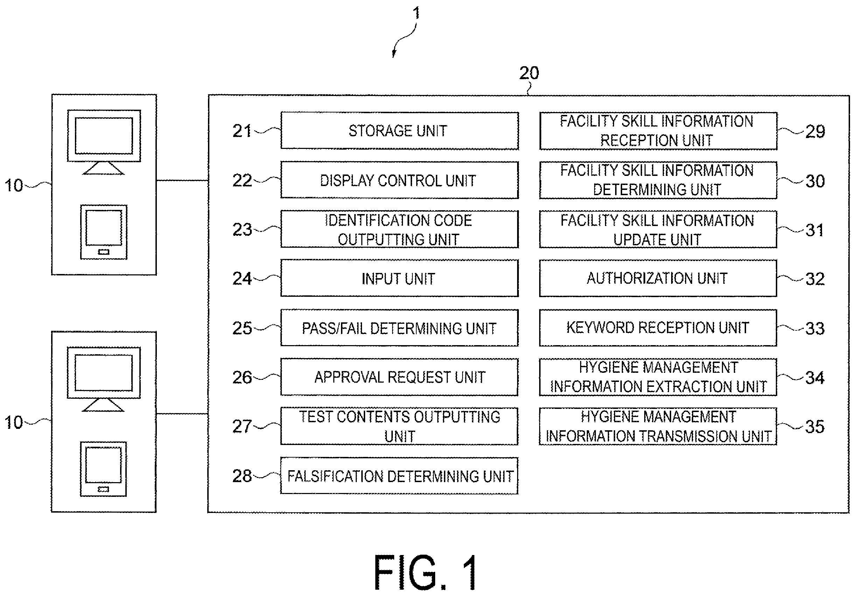

FIG. 1 is a view illustrating a functional configuration of the exemplary hygiene management system 1. The hygiene management system 1 includes, for example, a server 20. The exemplary server 20 is capable of communicating with each of terminals 10 of a plurality of facilities (for example, testers). In the present disclosure, the "tester" refers to a person that tests a specimen. The "terminal" includes an information terminal such as a personal computer and a portable terminal. The portable terminal includes a terminal capable of being carried on a person, such as a laptop computer, a tablet, or a mobile phone.

The server 20 includes, as functional components, a storage unit 21, a display control unit 22, an identification code outputting unit 23, an input unit 24, a pass/fail determining unit 25, an approval request unit 26, a test contents outputting unit 27, a falsification determining unit 28, a facility skill information reception unit 29, a facility skill information determining unit 30, a facility skill information update unit 31, an authorization unit 32, a keyword reception unit 33, a hygiene management information extraction unit 34, and a hygiene management information transmission unit 35.

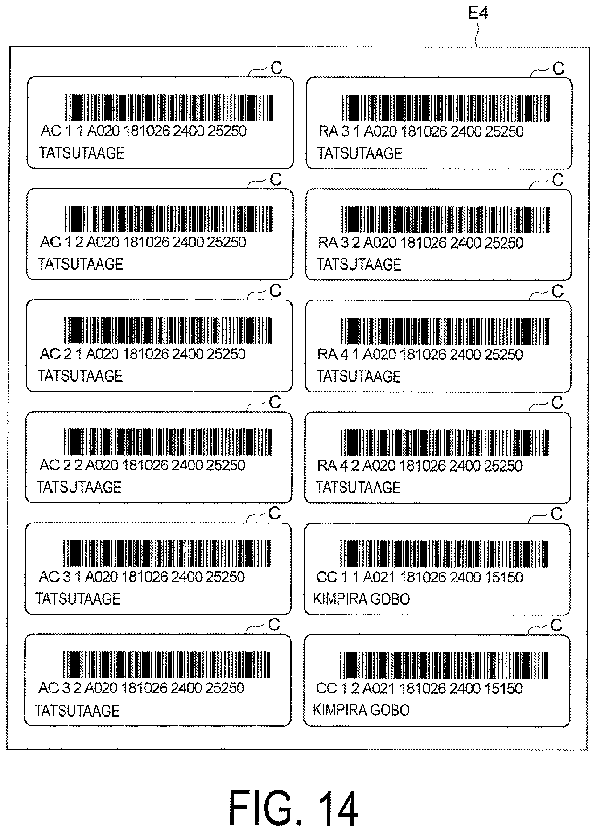

The storage unit 21 stores information received by the server 20 from the terminal 10 and information generated within the server 20. The storage unit 21 stores hygiene management information of a plurality of facilities in a database. The display control unit 22 controls a screen displayed on the terminal 10. The identification code outputting unit 23 outputs an identification code C (see FIG. 14) described below. The input unit 24 receives an input of a bacteria count (the number of test target substances) tested.

The pass/fail determining unit 25 determines a pass/fail for a bacteria count. The pass/fail determining unit 25 determines, for example, a pass/fail for a bacteria count by comparing a bacteria count input to the input unit 24 and a threshold value output in the identification code C. The pass/fail determining unit 25 may determine a fail when a bacteria count input to the input unit 24 is equal to or greater than a threshold value, and may determine a pass when a bacteria count input to the input unit 24 is less than a threshold value. The approval request unit 26 functions to request for approval to ship a specimen for which a pass/fail has been determined. The test contents outputting unit 27 functions to output test contents, and outputs, for example, a list of specimens to be tested for a bacteria count. The falsification determining unit 28 determines whether a bacteria count data file described below has been falsified.

The facility skill information reception unit 29 receives facility information and skill information. The facility skill information reception unit 29 may be a functional element that receives equipment information of a facility or may be a functional element that receives skill information of a tester in a facility. The facility information may refer to, for example, information of testing equipment of a facility, and may include a hygienic level and adequacy of testing equipment. The facility information may be an indicator that indicates whether a facility has the ability to produce accurate test results. The facility information may be ISO 17025 accreditation information. ISO 17025 is a management system for accreditation of accuracy of a laboratory and a calibration institute. ISO 17025 accreditation information is acquired to ensure compatibility with a QMS (quality management system), and ensure compatibility relating to the technical ability as a testing facility or a calibration facility.

The skill information may include a level of external accuracy management. The "external accuracy management" refers to a skill test or the like that indicates a skill for testing. Examples of the "external accuracy management" include a food analysis laboratory comparison (skill test) program, a microorganism test laboratory comparison (skill test) program, a genetic recombinant food qualitative/quantitative analysis laboratory comparison (skill test) program, and a fat analysis laboratory comparison (skill test) program. The facility skill information reception unit 29 receives, for example, a level (test results, for example, a Z score) of the above-described skill test from a facility, and uses the received level in pass/fail determination of the facility.

The facility skill information determining unit 30 is a functional element that determines the facility information and the skill information received by the facility skill information reception unit 29. The determination results by the facility skill information determining unit 30 are stored in the storage unit 21. The facility skill information determining unit 30 determines a pass/fail of each facility for the facility information and the skill information. The facility skill information determining unit 30 determines, for example, a pass/fail for the facility information and a pass/fail for the skill information. For example, in the facility skill information determining unit 30, a facility having acquired ISO 17025 accreditation information and having a level of external accuracy management equal to or greater than a certain value is determined to pass, and a facility not having acquired ISO 17025 accreditation information and having a level of external accuracy management less than the certain value is determined to fail.

The facility skill information update unit 31 is a functional element that periodically updates the facility information and the skill information of a facility stored in the storage unit 21. The facility skill information update unit 31 request a facility to update the facility information and the skill information in a certain amount of time after the facility information and the skill information have been stored in the storage unit 21.

The authorization unit 32 is a functional element that authorizes a facility to share hygiene management information with other facility different from the facility. The authorization unit 32 authorizes a facility determined to have passed by the facility skill information determining unit 30 to view hygiene management information of other facility and/or be viewable by other facility. The "authorizing to view hygiene management information of other facility and/or be viewable by other facility" refers to, for example, authorizing other facility to view hygiene management information of the own facility, and (in exchange for enabling this) authorizing the own facility to view hygiene management information of other facility. In this way, the authorization unit 32 enable a facility to view hygiene management information of other facility and/or enables other facility to view hygiene management information of the facility. For example, the authorization unit 32 enables a facility to view the hygiene management information of other facility, in exchange for enabling other facility to view hygiene management information of the facility. Such authorization is preferable in terms of a mutual advantage between facilities. In the following description and the drawings, the case of giving such mutual viewing authorization will be described by way of example. Hereinafter, this "mutual viewing authorization" may simply be described as "authorization". Note that the authorization unit 32 enable a facility to view hygiene management information of other facility, but, for example, does not enable the facility to view of which facility the hygiene management information is. That is, the authorization unit 32 does not specify the origin of hygiene management information. Enabling the management of one facility to view information from one or more other facilities may improve resource usage and/or the quality of hygiene management at the first facility. In one use case scenario, management of the first facility may determine that one or more other facilities are maintaining certain hygiene standards while expending fewer cleaning resources than the first facility. In this example, management of the first facility can reduce resource usage with respect to cleaning, while maintaining adequate hygiene standards at the first facility. By reducing the frequency of cleaning (and post-clean auditing) at the first facility, the management of the first facility may conserve the usage of potentially costly cleaning supplies and other inspection equipment, such as bioluminescence swabs and luminometers used for adenosine triphosphate (ATP) detection to implement indirect detection of microorganisms on surfaces and/or equipment. Microorganism detection and other types of hygiene monitoring cannot be effectuated with the unaided human eye due to the microscopic or nanoscopic form factors of the target analytes, so facility management relies on relatively sophisticated inspection equipment, such as bioluminescence assays, to perform hygiene monitoring and cleaning audits. The inspection equipment typically requires both consumables, such as single-use swabs for collecting samples and chemicals that are consumed in the analysis of samples, as well as hardware, such as luminometers, that can consume energy and operator time. By enabling facility management to minimize the amount of consumables that need to be used and usage of luminometers, as well as to mitigate network bandwidth consumption for uploading data more frequently, the systems of this disclosure may reduce the overall resource footprint of hygiene monitoring at various facilities while enabling facilities management to remain compliant with relevant hygiene standards. While described with respect to the example of the luciferase reaction-based equipment used for bioluminescence assays, it will be appreciated that the resource reduction benefits of this disclosure also apply to other hygiene monitoring mechanisms, such as indicator testing (where test are carried out for specific microorganisms that serve as indicators of a potential hygiene issue), which may be conducted by culture plate-based systems or other systems. In other use case scenarios, by enabling management of a first facility to access and view information submitted by one or more other facilities, the systems of this disclosure may improve the precision of the hygiene maintenance at the first facility, and thereby, compliance of the first facility with various hygiene standards. For example, if management of the first facility learns that management of one or more other facilities used to perform cleaning at the same frequency as the first facility, but later transitioned to a more frequent cleaning schedule, then the management of the first facility may determine that cleaning procedures/equipment are subpar, that the auditing equipment used at the first facility is potentially faulty, or that potentially useful audit information is otherwise being overlooked at the first facility. In these examples, the systems of this disclosure may enable the management of the first facility to improve the cleaning procedures or otherwise fine-tune hygiene maintenance at the first facility to be at least at par with the peer facilities. In these examples, the systems of this disclosure provide error resilience and precision improvements with respect to hygiene monitoring and compliance using information that is sourced from multiple facilities. In various examples, the facilities participating in the cross-facility information sharing techniques of this disclosure may be commonly owned, may be under shared administration, or may, whether related or not in operation, be voluntary participants in a hygiene management information sharing program implemented using the systems of this disclosure. Additionally, the cross-facility information sharing-based hygiene management techniques of this disclosure are sufficiently scalable to accommodate testing for specific analytes as well as to accommodate more coarse-grained inspection techniques that test for the mere presence of live organism(s) on a surface or in equipment.

The keyword reception unit 33 is a functional element that receives a search keyword for searching for hygiene management information of other facility. The keyword reception unit 33 receives from a facility (for example, from the terminal 10) a search keyword for searching for hygiene management information. The search keyword received by the keyword reception unit 33 may include, for example, a character string relating to at least any of testing standards, a test object, test contents, and a testing method for a test target substance.

The hygiene management information extraction unit 34 is a functional element that extracts hygiene management information stored in the storage unit 21 by using a search keyword received by the keyword reception unit 33. The hygiene management information extraction unit 34 extracts from the storage unit 21 hygiene management information that matches a search keyword. The hygiene management information extraction unit 34 extracts from the storage unit 21, for example, a plurality of pieces of hygiene management information tagged with a search keyword.

The hygiene management information transmission unit 35 is a functional element that transmits hygiene management information extracted by the hygiene management information extraction unit 34. That is, the hygiene management information transmission unit 35 transmits to a facility (to the terminal 10, for example) hygiene management information extracted by the hygiene management information extraction unit 34. The hygiene management information transmission unit 35 may transmits hygiene management information to one facility or may transmit hygiene management information to a plurality of facilities.

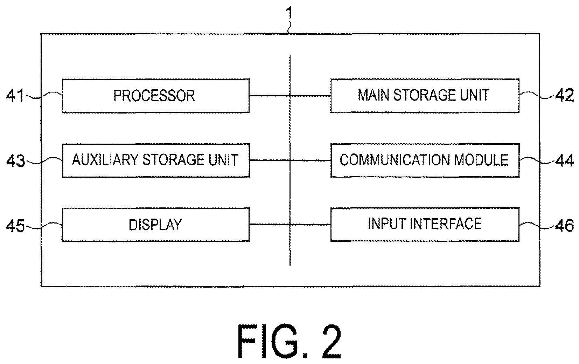

FIG. 2 is a view illustrating an example of a hardware configuration of the hygiene management system 1 (for example, the terminal 10 and the server 20). For example, each of the above-described functional elements of the server 20 is realized by this hardware configuration. For example, the server 20 includes a processor 41, a main storage unit 42, an auxiliary storage unit 43, a communication module 44, a display 45, and an input interface 46. The processor 41 is a computation device that executes an operating system and an application program. The main storage unit 42 includes, for example, a ROM or a RAM, and temporarily stores a loaded program, computation results, or the like. The auxiliary storage unit 43 includes a flash memory, a hard disk, or the like and permanently stores a program or data.

The communication module 44 includes a wireless communication module or a network card, and transmits and receives data to and from other computer. The display 45 includes a touch panel or a monitor and is a device that displays data or information to be visually recognizable by a user. The input interface 46 includes a touch panel, a mouse or the like, and is a device that receives an input of data or an instruction by a user.

For example, each of the above-described functional elements of the server 20 is realized by executing predetermined software (for example, the hygiene management program described above) loaded onto the processor 41 or the main storage unit 42. According to the software, the processor 41 operates the communication module 44, the display 45, or the input interface 46, and reads and writes data in the main storage unit 42 or the auxiliary storage unit 43. Data or the like in a database is stored in the main storage unit 42 or the auxiliary storage unit 43.

As described above, the server 20 may include a single computer or may include a plurality of computers. For example, in the case of the server 20 including a plurality of computers, the plurality of computers are connected each other via a communication network such as the Internet or an intranet, and thus logically constitute the single server 20.

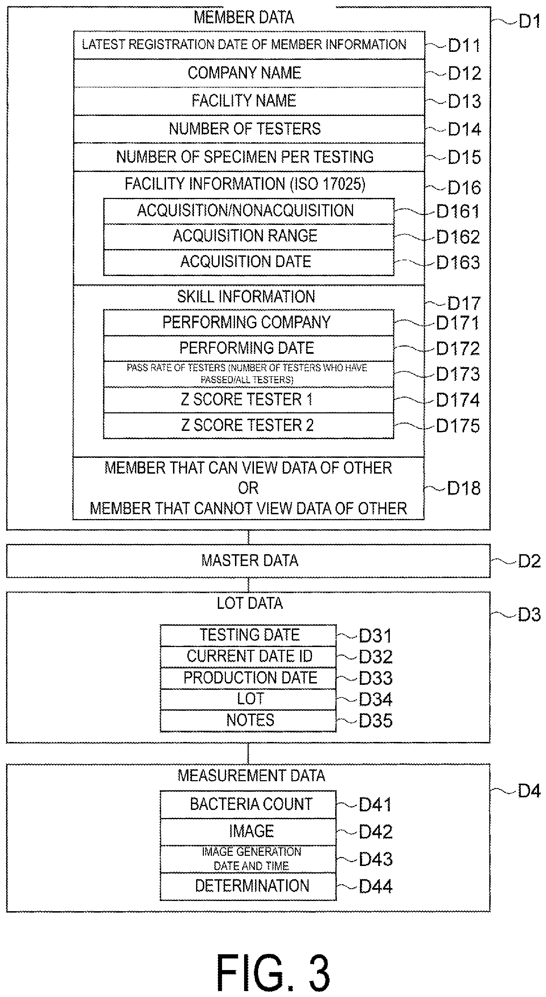

FIG. 3 is a view illustrating a configuration of data stored in the storage unit 21. As illustrated in FIG. 3, member data D1, master data D2, lot data DO, and measurement data D4 are stored in the storage unit 21. In the present embodiment, the "member" refers to a kind of "facility." The member data D1 includes, for example, the latest registration date of member information D11, a company name D12, a facility name D13, the number of testers D14, the number of specimens per testing D15, facility information D16, skill information D17, and allowance of viewing data of other D18.

The facility information D16 includes, for example, data relating to acquisition/nonacquisition of ISO 17025, and includes, for example, acquisition/nonacquisition D161, the acquisition range D162, the acquisition date D163. The skill information D17, for example, includes a company that has performed external accuracy management D171, the performing date D172, a pass rate of testers D173, and a Z score D174, D175 of each tester. The pass rate of testers D173 indicates a proportion of the number of testers having passed an external accuracy management test to the total number of testers in a facility. The allowance of viewing data of other D18 indicates whether a facility is a member that can view data of other, or whether a facility is a member that cannot view data of other. The "member that can view data of other" refers to a facility that can view hygiene management information of other facility. The "member that cannot view data of other" refers to a facility that cannot view hygiene management information of other facility.

The lot data D3 includes, for example, the specimen testing date D31, current date ID D32, the production date D33, a lot D34, and notes D35. The measurement data D4 includes a bacteria count D41 (the number of test target substances), an image D42 (image data), the image generation date and time D43, and determination D44 (pass/fail determination results for a specimen).

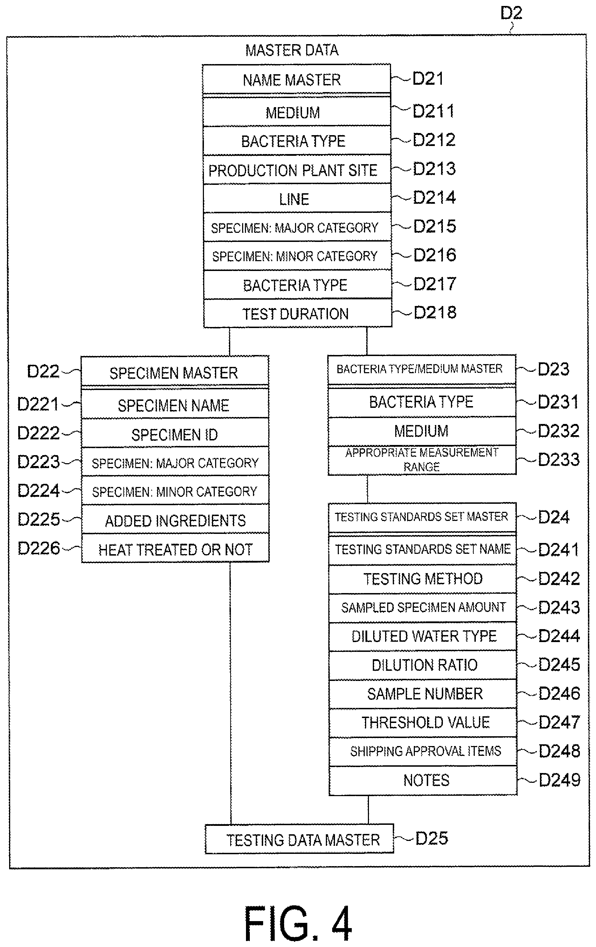

FIG. 4 is a view illustrating the master data D2 in detail. As illustrated in FIG. 4, the master data D2 includes a name master D21, a specimen master D22, a bacteria type/medium master D23, a testing standards set master D24, and a testing data master D25.

The name master D21 includes, for example, a medium D211 (a type of medium, for example), a bacteria type D212 (a type of test target substance such as "viable bacteria"), a production plant site D213, a line D214, a specimen: major category D215, a specimen: minor category D216, a bacteria type D217, and test duration D218.

The specimen master D22 includes a specimen name D221 (food name such as "frozen fried chicken", for example), specimen ID D222, a specimen: major category D223, a specimen: minor category D224, added ingredients D225, and heat treated or not D226.

The bacteria type/medium master D23 includes a bacteria type D231, a medium D232, and the appropriate measurement range D233. The "appropriate measurement range" is, for example, the numerical range for determining whether a bacteria count detected by a plate described below is adopted or rejected.

For example, the "testing standards set" refers to test contents such as a "routine test". The testing standards set master D24 includes a testing standards set name D241, a testing method D242, a sampled specimen amount D243, a diluted water type D244, a dilution ratio D245, the sample number D246 (for example, the number of plates described below), a threshold value D247, shipping approval items D248, and notes D249.

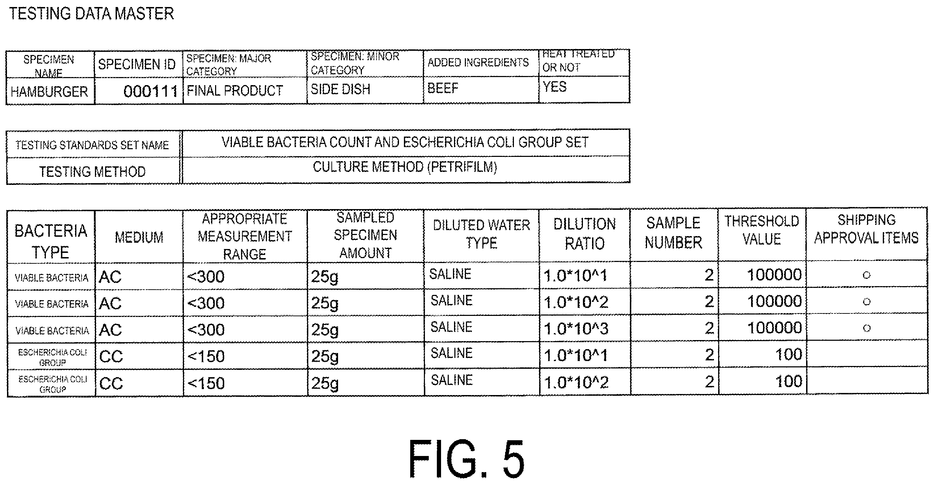

FIG. 5 illustrates a specific example of data items of the testing data master D25. The testing data master D25 includes information of the specimen master D22, the bacteria type/medium master D23, and the testing standards set master D24. As illustrated in FIG. 5, for example, the testing data master D25 includes the specimen name D221, the specimen ID D222, the specimen: major category D223, the specimen: minor category D224, the added ingredients D225, the heat treated or not D226, the testing standards set name D241, the testing method D242, the bacteria type D231, the medium D232, the appropriate measurement range D233, the sampled specimen amount D243, the diluted water type D244, the dilution ratio D245, the sample number D246, the threshold value D247, and the shipping approval items D248.

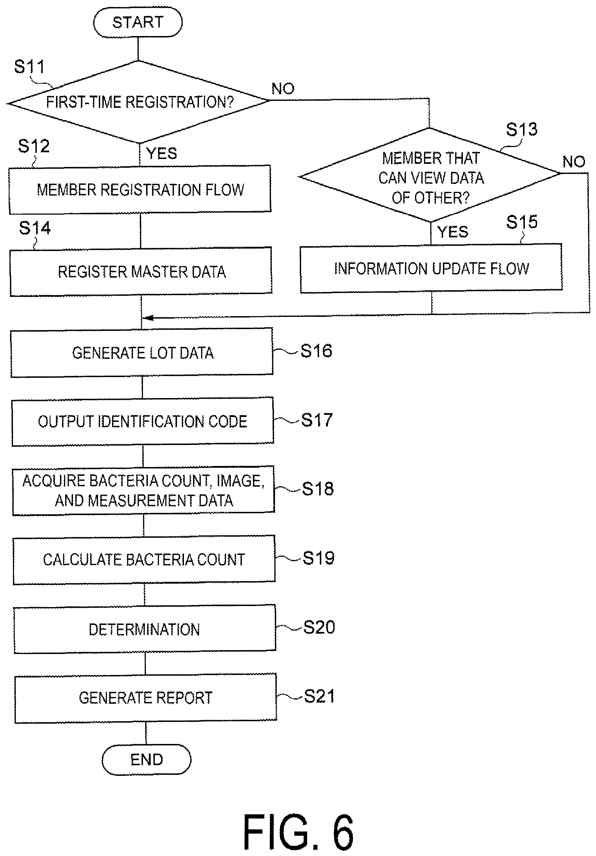

FIG. 6 is an exemplary flowchart of performing calculation of a bacteria count and determination of a bacteria count of a specimen, and the calculation and the determination are executed by the hygiene management system 1, the hygiene management method, and the hygiene management program according to an embodiment. In the hygiene management system 1, the hygiene management method, and the hygiene management program according to the present embodiment, for example, handwritten work can be made unnecessary at steps S11 to S21 described below, and efficient specimen testing can be made. For example, the following steps are executed by the server 20 of the hygiene management system 1.

First, it is determined whether a facility is a first-time registration facility (step S11). At step S11, when a facility is a first-time registration facility (when a facility is a facility to be registered for the first time), the process proceeds to step S12. When it is determined that the facility is not a first-time registration facility, the process proceeds to step S13.

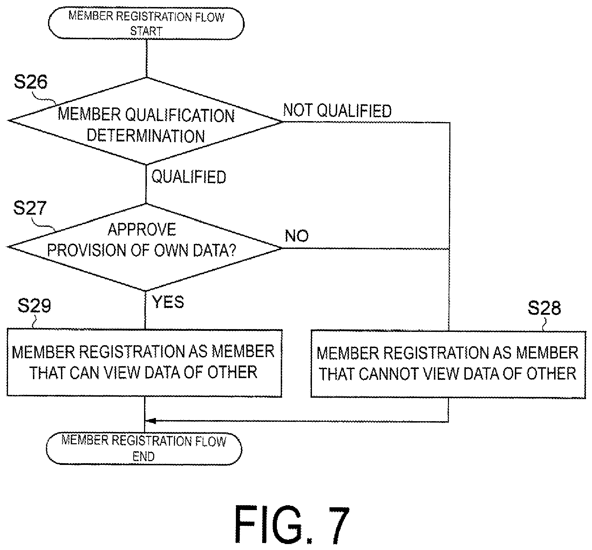

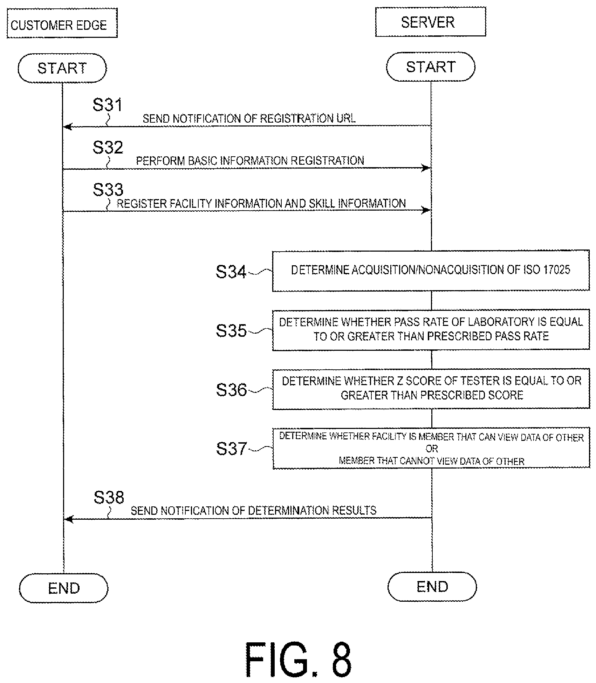

At step S12, for example, a member registration flow illustrated in FIG. 7 and FIG. 8 is executed. In the member registration flow, first, member qualification determination is performed (step S26). As illustrated in FIG. 8, in the member qualification determination, for example, registration URL notification is sent to the terminal 10 (customer edge) from the server 20.

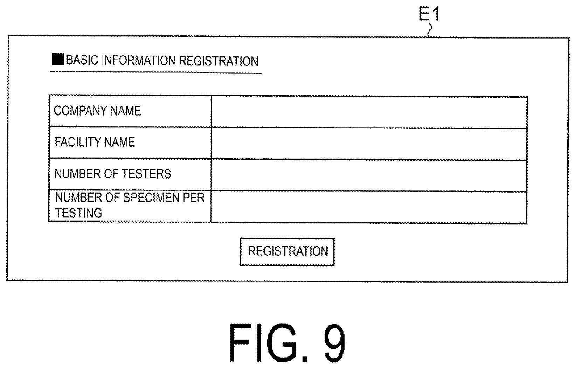

When the registration URL is tapped on the terminal 10, a basic information registration screen E1 illustrated in FIG. 9 is displayed on the terminal 10. On the basic information registration screen E1, for example, when the company name, the facility name, the number of testers, and the number of specimens (per testing) are input and a registration button is pressed, basic information registration is performed (step S32).

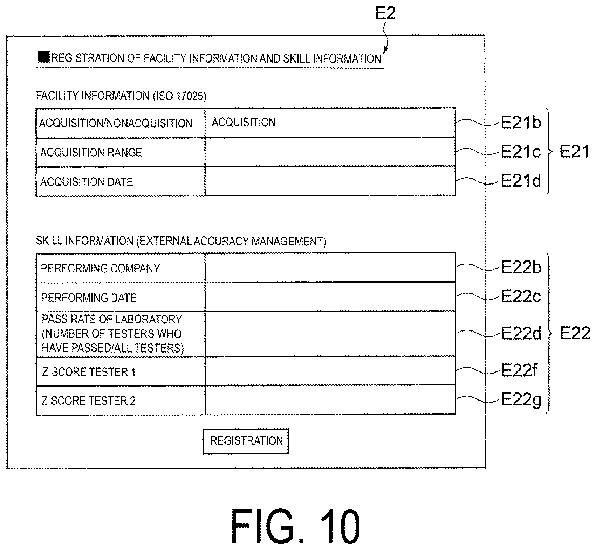

Next, a registration screen E2 for facility information and skill information illustrated in FIG. 10 is displayed on the terminal 10. The registration screen E2 includes a facility information input unit E21 and a skill information input unit E22. The facility information input unit E21 includes, for example, ISO 17025 acquisition/nonacquisition E21b, the acquisition range E21c, and the acquisition date E21d as items. The skill information input unit E22, for example, includes a company that has performed external accuracy management E22b, the performing date E22c, a pass rate of testers E22d, and a Z score E22f, E22g of each tester as items.

On the registration screen E2, when data is input in the items of the facility information input unit E21 and the items of the skill information input unit E22 and a registration button is pressed, the facility information and the skill information is transmitted to the server 20 (the step of transmitting facility information and skill information). The transmitted facility information and skill information are received by the facility skill information reception unit 29 (the step of receiving from a facility, the step of receiving from a facility), and the facility information and the skill information are registered (step S33).

Subsequently, as illustrated in FIG. 8, for example, the facility skill information determining unit 30 performs ISO 17025 acquisition/nonacquisition determination (step S34), determination of whether a pass rate of testers in an external accuracy management test is equal to or greater than a prescribed pass rate (step S35), and determination of whether a Z score of each tester in an external accuracy management test is equal to or greater than a prescribed score (step S36).

Then, the facility skill information determining unit 30 determines whether a facility having performed various kinds of registration on the terminal 10 is a viewable member or a non-viewable member (the step of determining a pass/fail of a facility, the step of determining a pass/fail of a facility, step S37). That is, the facility skill information determining unit 30 determines whether a facility is qualified to be a viewable member. For example, the facility skill information determining unit 30 determines that a facility is a viewable member when the facility is determined, at step S34, to have acquired ISO 17025, determined, at step S35, to have a pass rate equal to or greater than a prescribed pass rate, and determined, at step S36, to have a Z score equal to or greater than a prescribed score.

For example, the facility skill information determining unit 30 determines that a facility is a non-viewable member when the facility is determined, at step S34, to have not acquired ISO 17025, determined, at step S35, to not have a pass rate equal to or greater than a prescribed pass rate, or determined, at step S36, to not have a Z score equal to or greater than a prescribed score. After the above-described determination has been made, notification of pass/fail determination results determined by the facility skill information determining unit 30 is send to the terminal 10 (the step of transmitting pass/fail results to a facility, the step of transmitting pass/fail results to the facility, step S38). The facility receives the pass/fail determination results by the terminal 10 (the step of receiving the determination results). Then, the authorization unit 32 authorizes the facility determined to be a viewable member (the step of authorizing to be viewable, the step of authorizing to be viewable), and a series of steps of member qualification determination ends.

As illustrated in FIG. 7, as a result of the member qualification determination, when the facility skill information determining unit 30 determines that the facility is qualified, the process proceeds to step S27. On the other hand, as a result of the member qualification determination, when the facility skill information determining unit 30 determines that the facility is not qualified, the process proceeds to step S28.

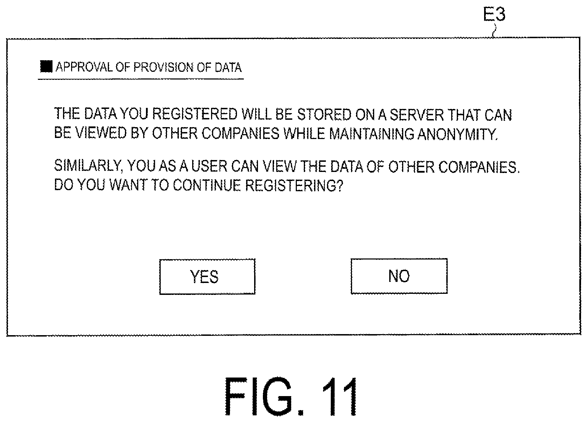

At step S27, it is determined whether a facility having received the determination results determined by the facility skill information determining unit 30 approves provision of data of the own facility (the facility) to the hygiene management system 1. Here, as illustrated in FIG. 11, a data provision approval screen E3 is displayed on the terminal 10. The data provision approval screen E3 corresponds to a screen requesting approval from the facility to provide data (hygiene management information) of the own facility. For example, when a button "YES" is pressed on the data provision approval screen E3, provision of data of the own facility is approved. When a button "NO" is pressed on the data provision approval screen E3, provision of data of the own facility is rejected.

When the facility rejects the provision of data of the own facility (NO at step S27), the facility is registered as a member that cannot view data of other (step S28). On the other hand, when the facility approves the provision of data of the own facility (YES at step S27), the facility is registered as a member that can view data of other (step S29). After step S28 or step S29 described above is completed, the member registration flow ends.

As illustrated in FIG. 6, after the member registration flow is completed, registration of the master data D2 is performed (step S14). Here, for example, the contents input on the basic information registration screen E1 are registered as the latest registration date of member information D11, the company name D12, the facility name D13, the number of testers D14, and the number of specimens D15 of the member data D1. Then, the contents input on the registration screen E2 are registered as respective items of the facility information D16 and the skill information D17 of the member data D1. Note that registration of the master data D2 may be performed together with the member registration flow (step S12) instead of after the member registration flow.

In FIG. 6, when it is determined that a facility is not a first-time registration facility (NO at step S11), the process proceeds to step S13 and it is determined whether the facility is a member that can view data of other. Here, when it is determined that the facility is a member that can view data of other, the process proceeds to an information update flow (step S15). When it is determined that the facility is not a member that can view data of other, the process proceeds to step S16.

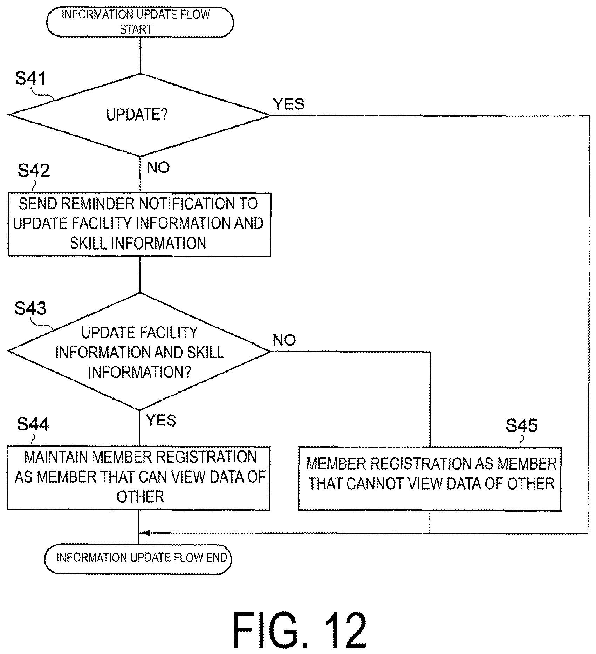

At step S15, for example, the information update flow illustrated in FIG. 12 is executed. In the information update flow, for example, it is determined whether the facility information and the skill information has been updated within a certain amount of time (for example, half a year or one year) (step S41). Here, when it is determined that the facility information and the skill information has been updated within a certain amount of time (YES at step S41), the information update flow ends. On the other hand, when it is determined that the facility information and the skill information has not been updated within a certain amount of time (NO at step S41), reminder notification to update the facility information and the skill information is send to the facility (for example, the terminal 10) (step S42).

In a predetermined amount of time after step S42 (for example, one or two months), it is determined whether the facility information and the skill information has been updated (step S43). Here, when it is determined that the facility information and the skill information has been updated (YES at step S43), the facility is maintained as a member that can view data of other (step S44). On the other hand, when it is determined that the facility information and the skill information has not been updated (NO at step S43), the facility is re-registered as a member that cannot view data of other (step S45). After step S44 or step S45 is completed, the information update flow ends.

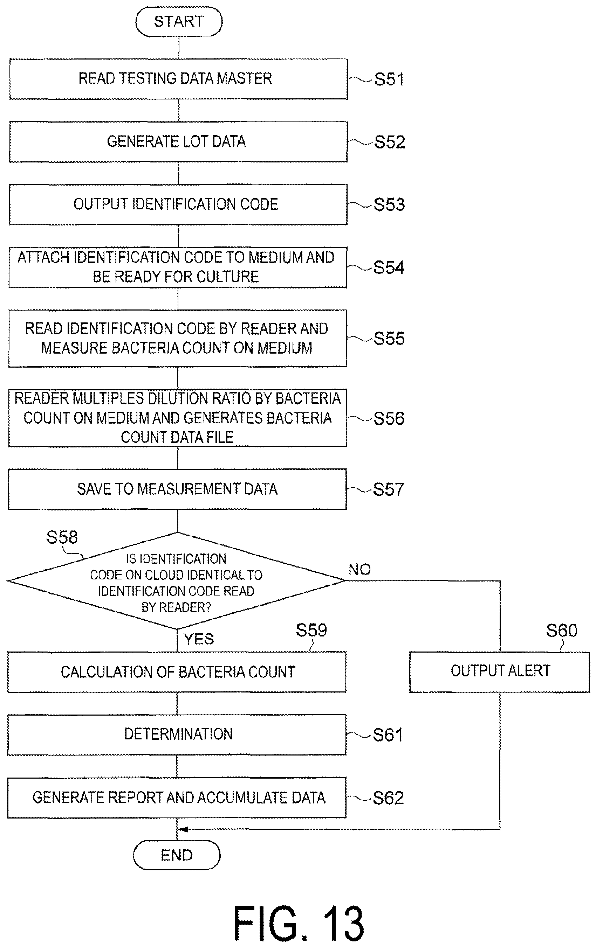

After the information update flow is completed, as illustrated in FIG. 13 for example, reading of the testing data master D25 is performed (step S51). In the reading of the testing data master D25, testing items for a specimen are extracted, for example. Note that FIG. 13 is a flowchart illustrating the steps of the flowchart of FIG. 6 in more detail. After the reading of the testing data master D25 is performed, the lot data D3 is generated (step S16, step S52). Here, for example, each item of the lot data D3 is input from the terminal 10 and each item of the lot data D3 (the specimen testing date D31, the current date ID D32, the production date D33, the lot D34, and the notes D35) is registered.

After the lot data D3 is generated, for example, as illustrated in FIG. 14, the identification code outputting unit 23 outputs the identification code C (step S17, step S53). The identification code C is displayed, for example, as an identification code display screen E4. The identification code display screen E4 includes, for example, the identification code C associated with a plate described below. The identification code C includes identification information for identifying a plate. In the present embodiment, the identification code C is a barcode. However, the identification code C is not limited to a barcode and may be other type of identification code such as a QR code (registered trademark).

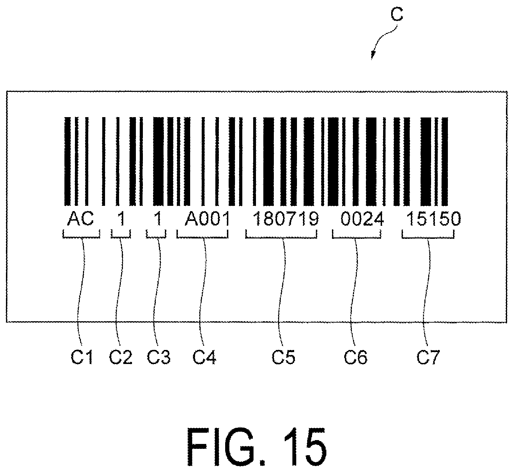

FIG. 15 is an enlarged view of the identification code C. As illustrated in FIG. 14 and FIG. 15, the identification code C includes identification information of a plate, for example, a medium C1 a dilution ratio C2, the sample number C3 (the number of plates), individual ID C4, the testing date C5, a threshold value C6, and the appropriate measurement range C7. In the present embodiment, the "threshold value" refers to a reference value for determining a pass/fail for an obtained bacteria count of a specimen. For example, when a bacteria count is equal to or greater than the threshold value, it is determined to be a fail, and when a bacteria count is less than the threshold value, it is determined to be a pass. The "appropriate measurement range" refers to, for example, the numerical range for determining whether a bacteria count detected by a plate is adopted or rejected.

Note that the identification code C does not need to include all of the medium C1, the dilution ratio C2, the sample number C3, the individual ID C4, the testing date C5, the threshold value C6, and the appropriate measurement range C7, and may further include other information. Further, a method of expressing the identification code C together with the contents present in the identification code C may be changed as appropriate. For example, instead of numbers described in the identification code C, letters such as alphabets may be used, or numbers of the identification code C may be changed to alphabets and the number of characters of the identification code C may be shortened.

Each identification code C illustrated in FIG. 14 may be printed out as a sticker provided in a predetermined printing medium, for example. In this case, when the printing medium is set in a printing machine such as a printer and the identification code display screen E4 is printed out, the printing medium is printed out in a state where the identification code C is printed in a sticker portion of the printing medium. The identification code C is peeled off from the printing medium printed out, and the identification code C peeled off is attached to a plate, and then the plate is ready for culture of a specimen (step S54).

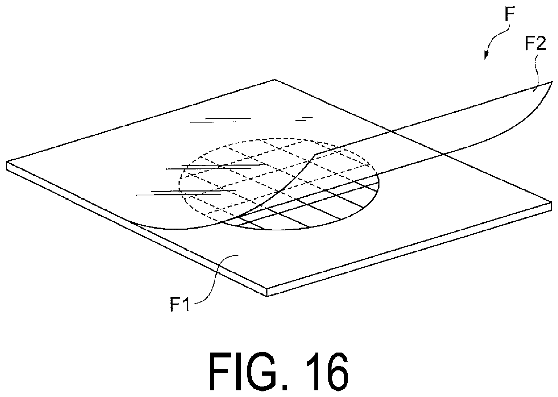

FIG. 16 is a perspective view illustrating an exemplary plate F to which the identification code C is attached. In the present embodiment, a bacteria count of a specimen is calculated by using the plate F. Note that in the related art, a test has been performed by using an agar medium. However, in a case where a test is performed by using the plate F as in the present embodiment instead of an agar medium, testing time can be reduced greatly. Then, the plate F is formed as a film. Thus, a space required for culture can be reduced greatly, and bacteria (also referred to as a colony) can be observed easily. Further, waste disposal after culture can be performed easily.

In the present embodiment, the "plate" refers to a sheet-like member including a medium for bacteria of a specimen, and is, for example, a tool on which a specimen diluted in a medium is placed to enable testing of a bacteria count of the specimen. For example, the plate F includes a lower film F1 on which a specimen is placed and an upper film F2 that covers the lower film F1. For example, the lower film F1 includes a foam dam, a medium component, an adhesive agent, and a plastic coating paper, and the upper film F2 includes a cold water-soluble gel, an indicator reagent-containing adhesive agent, and a plastic film. For example, a specimen (test solution) is placed in the center of the lower film F1, and the upper film F2 covers the specimen and the lower film F1. Then, when a spreader (not illustrated) is placed on the upper film F2 to spread the specimen, the specimen gels in a certain amount of time (for example, in one minute).

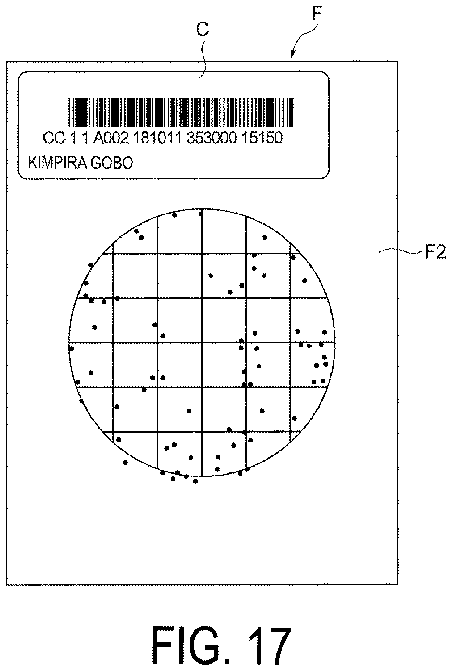

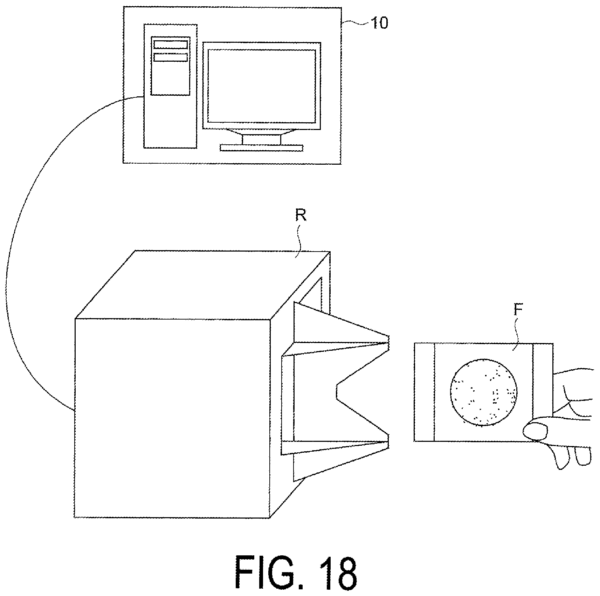

Then, after the specimen is cultured for a predetermined amount of time, spots of bacteria of the specimen develop, for example, as illustrated in FIG. 17 and FIG. 18. After the identification code C is attached to the plate F on which the bacteria are developed in this way, the plate F to which the identification code C is attached is inserted to a reader R. The reader R is an apparatus that reads a bacteria count by counting the number of bacteria developed on the plate F. In the related art, a bacteria count on the plate F has been counted manually and has been inefficient. However, in the case of the reader R, a bacteria count can be counted in a short amount of time (for example, approximately four seconds) by inserting the plate F to the reader R. For example, the reader R automatically counts a bacteria count on the plate F and outputs the counted bacteria count in a file format.

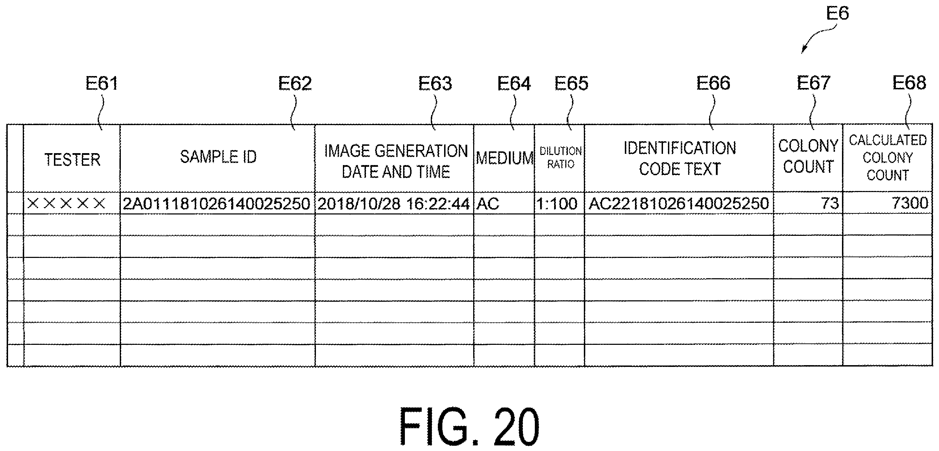

As illustrated in FIG. 18, FIG. 19, and FIG. 20, when the plate F is inserted to the reader R, for example, the reader R reads the plate F, and software installed in the terminal 10 capable of communicating with the reader R measures a bacteria count (step S18, step S55). Then, a bacteria count data screen E5 is output on the terminal 10 and also the bacteria count is calculated, and a bacteria count data file E6 including the calculated bacteria count is output (step S19, step S56).

The bacteria count data screen E5 includes, for example, a plate screen E51, a sample ID E52, a medium E53, a dilution ratio E54, a colony count E55 (bacteria count), and a calculated colony count E56. For example, in a case where the bacteria count data file E6 is selected to be output in previous setting, the bacteria count data file E6 is automatically output. The bacteria count data file E6 includes a tester E61, a sample ID E62, an image generation date and time E63, a medium E64, a dilution ratio E65, an identification code text E66, a colony count E67, and a calculated colony count E68.

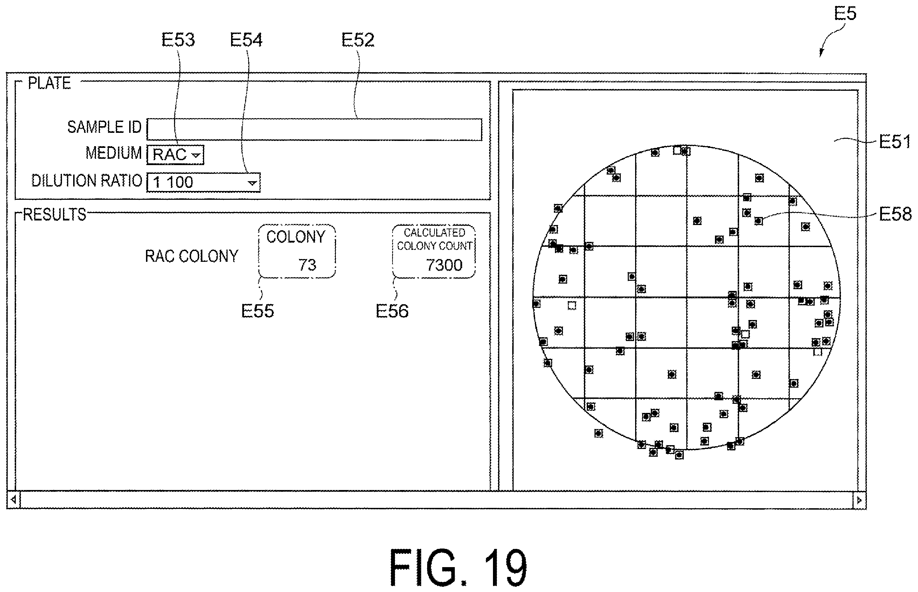

The plate screen E51 displays, for example, the plate F together with a mark E58 for highlighting a position of a colony. In the example of FIG. 19, the mark E58 having a rectangular shape is displayed to surround a colony roundly displayed. For example, the sample ID E52, E62 and the identification code text E66 together include identification information for identifying the plate F, and the identification code text E66 may indicate text data of the identification code C attached to the plate F. The colony count E55, E67 indicates the number of colonies displayed on the plate screen E51, and the calculated colony count E56, E68 is a numerical value indicating a product of the colony count displayed on the plate screen E51 and a dilution ratio.

In the present embodiment, for example, the bacteria count data file E6 is uploaded to the master data D2. That is, the bacteria count data file E6 is stored on the cloud (storage unit 21) (step S57). Subsequently, for example, the falsification determining unit 28 may determine whether the bacteria count data file E6 has been falsified. Note that the falsification determining unit 28 may be omitted. For example, the falsification determining unit 28 determines whether the identification code C on the cloud and the identification code C read are the same (step S58).

For example, when the falsification determining unit 28 determines that the identification code C on the cloud and the identification code C read are the same, a bacteria count is calculated (step S19, step S59). On the other hand, when the falsification determining unit 28 determines that the identification code C on the cloud and the identification code C read are not the same, an alert is output (step S60). At step S60, for example, an alert notifying that the bacteria count data file E6 has been falsified may be output. After the alert is output, a series of steps may end.

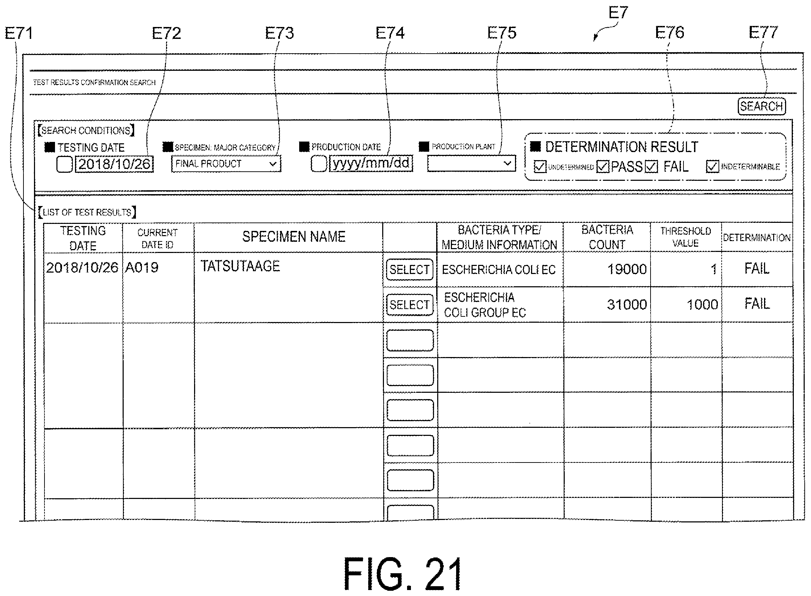

In the calculation of a bacteria count, for example, a test results confirmation screen E7 illustrated in FIG. 21 is displayed. The test results confirmation screen E7 is displayed, for example, on the terminal 10. The test results confirmation screen E7 includes, for example, a test results display unit E71, a testing date input unit E72, a specimen: major category selection unit E73, a production date input unit E74, a production plant selection unit E75, a determination results selection unit E76, and a search button E77. In the determination results selection unit E76, for example, any of undetermined, a pass, a fail, and indeterminable is selectable. For example, in the determination results selection unit E76, by default, all of undetermined, a pass, a fail, and indeterminable are selected.

For example, when the search button E77 is pressed in a state where the testing date input unit E72 and the determination results selection unit E76 are input, test results on the testing date input in the testing date input unit E72 are displayed on the test results display unit E71. Note that in a case where any of the specimen: major category selection unit E73, the production date input unit E74, the production plant selection unit E75, and the determination results selection unit E76 is selected as a condition, for example, test results satisfying the selected condition are only displayed on the test results display unit E71.

The test results display unit E71 displays, for example, the testing date, the current date ID, the specimen name, a selection button, bacteria type/medium information, a bacteria count, a threshold value, and determination results. The testing date of the test results display unit E71 matches the testing date input in the testing date input unit E72. The current date ID of the test results display unit E71 is, for example, an ID that matches the individual ID C4 of the identification code C and indicates the ID assigned to the day of the testing date. When the selection button of the test results display unit E71 is pressed, a screen of the detail of test results is displayed, for example. The bacteria type/medium information of the test results display unit E71 includes, for example, the bacteria type D231 and the medium D232 stored in the bacteria type/medium master D23 described above.

The determination displayed on the test results display unit E71 indicates, for example, a pass/fail for a value of a bacteria count obtained by calculating, in accordance with a predetermined rule, the colony count E68 of the bacteria count data file E6 that is a bacteria count obtained at each dilution stage. The determination of a pass/fail for a bacteria count on the test results display unit E71 is performed by, for example, the pass/fail determining unit 25 described above (step S20, step S61). For example, when a bacteria count is equal to or greater than the threshold value C6, the pass/fail determining unit 25 determines a fail, and when a bacteria count is less than the threshold value C6, the pass/fail determining unit 25 determines a pass.

After the determination of a bacteria count is performed as described above, a test report is generated (step S21, step S62). In the generation of a report, for example, a report is generated by performing an input on a formatted test report automatically displayed. At this time, data analysis and accumulation may be performed. For example, each item of the measurement data D4 obtained as a result of testing is updated. As a specific example, a bacteria count is stored in the bacteria count D41, the plate screen E51 is stored in the image D42 of the measurement data D4, the generation data of the plate screen E51 is stored in the image generation date and time D43, and the determination results of the bacteria count described above is stored in the determination D44.

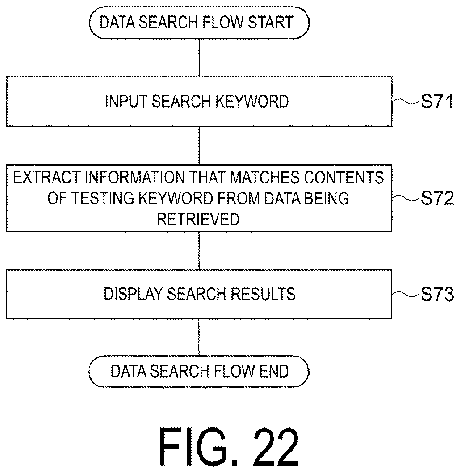

The above-described testing, and the above-described update of each item of the measurement data D4 are performed, for example, in each of the plurality of facilities. Next, an example of each step (viewing step) of a flow where a facility that is a member that can view data of other views hygiene management information of other facility will be described with reference to a flowchart of FIG. 22. The data search flow of FIG. 22 is performed, for example, by the terminal 10 described above.

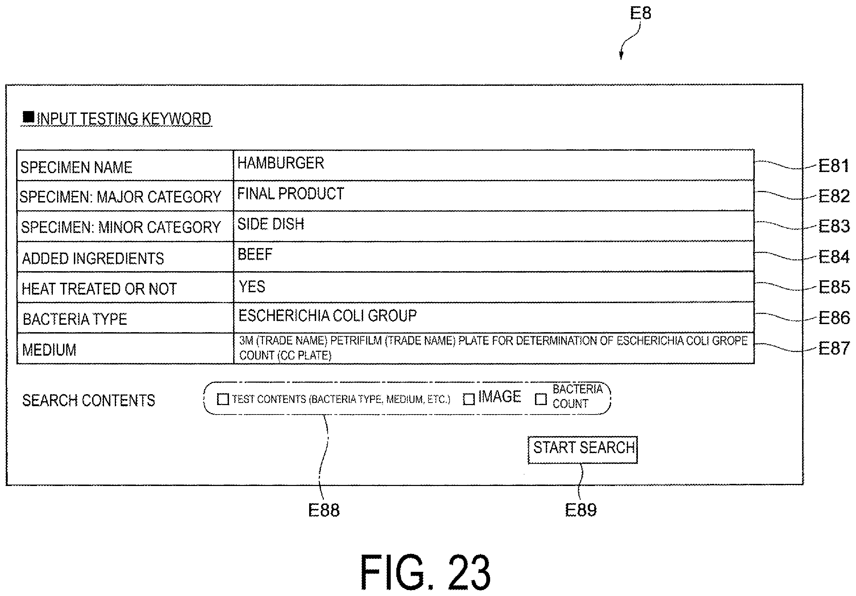

For example, when software installed in the terminal 10 is activated, an input screen E8 for a search keyword illustrated in FIG. 23 is displayed. First, a search keyword is input on the input screen E8 (step S71). Specific examples of the input include an input of at least any of a specimen name E81, a specimen: major category E82, a specimen: minor category E83, added ingredients E84, heat treated or not E85, a bacteria type E86, and a medium E87.

Then, In a check box group E88 of search contents, a check box of contents to be searched for is checked. The check box group E88 includes, for example, check boxes of test contents (a bacteria type, a medium, and the like), an image, and a bacteria count. Among these check boxes, the check box of contents to be searched for is checked. Then, the input of the search keyword is completed by pressing a search start button E89.

When the search keyword is input, the input keyword is received by the keyword reception unit 33. Then, the hygiene management information extraction unit 34 extracts, from the master data D2 or the measurement data D4, hygiene management information tagged with the input keyword (for example, at least any of the specimen name E81, the specimen: major category E82, the specimen: the minor category E83, the added ingredients E84, the heat treated or not E85, the bacteria type E86, and the medium E87) (step S72).

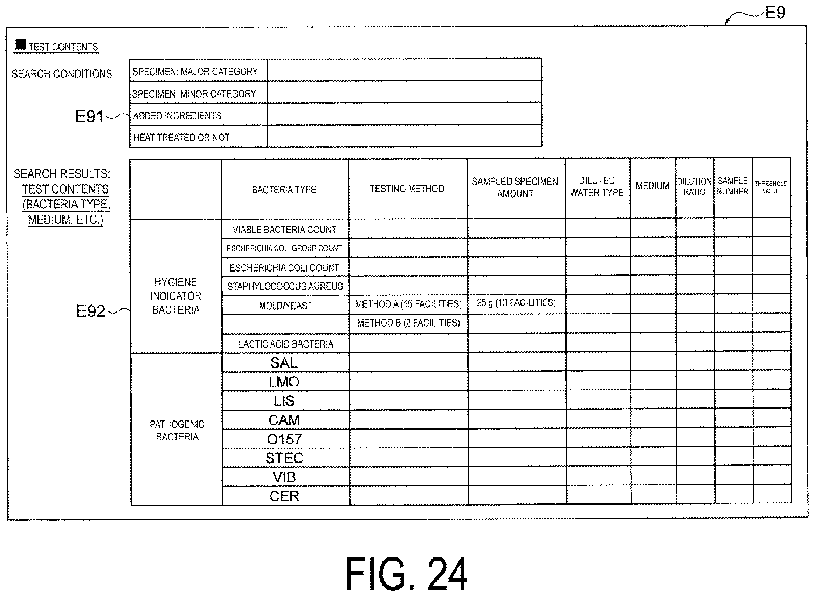

The hygiene management information transmission unit 35 transmits the hygiene management information extracted by the hygiene management information extraction unit 34 to the terminal 10, and, for example, search results of the hygiene management information are displayed on the terminal 10 (step S73). FIG. 24 illustrates a test contents screen E9 that is an example of the hygiene management information of other facility that is displayed. The test contents screen E9 is a screen that is displayed when the check box of the test contents (a bacteria type, a medium, and the like) is checked in the check box group E88 of the input screen E8.

The test contents screen E9 includes, for example, a search condition E91 and search results E92. In the search condition E91, at least any of the specimen: major category E82, the specimen: minor category E83, the added ingredients E84, and the heat treated or not E85 input on the input screen E8 is displayed. For example, in the search results E92, a testing method, a sampled specimen amount, a diluted water type, a medium, a dilution ratio, the sample number, a threshold value tagged with the item input on the input screen E8 are displayed for each bacteria type. The data displayed in the search results E92 corresponds to information of the testing standards set master D24 tagged with the bacteria type D231 of the bacteria type/medium master D23.

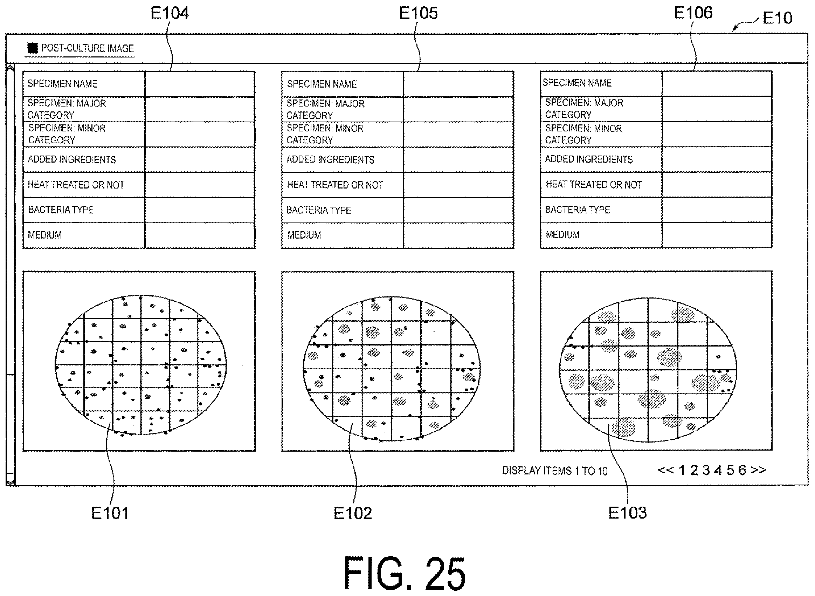

FIG. 25 illustrates a post-culture image data E10 that is another example of the hygiene management information of other facility that is displayed. The image data E10 includes, for example, post-culture images E101, E102, E103, and search conditions E104, E105, E106. In the search conditions E104, E105, E106, at least any of the specimen name E81, the specimen: major category E82, the specimen: minor category E83, the added ingredients E84, the heat treated or not E85, the bacteria type E86, and the medium E87 input on the input screen E8 is displayed. The images E101, E102, and E103 are images that are tagged with the items (for example, a bacteria type) input on the input screen E8. The images E101, E102, E103 correspond to the image D42 of the measurement data D4, for example. Note that the image generation date and time D43 may be displayed together with the images E101, E102, and E103 as the image data E10.

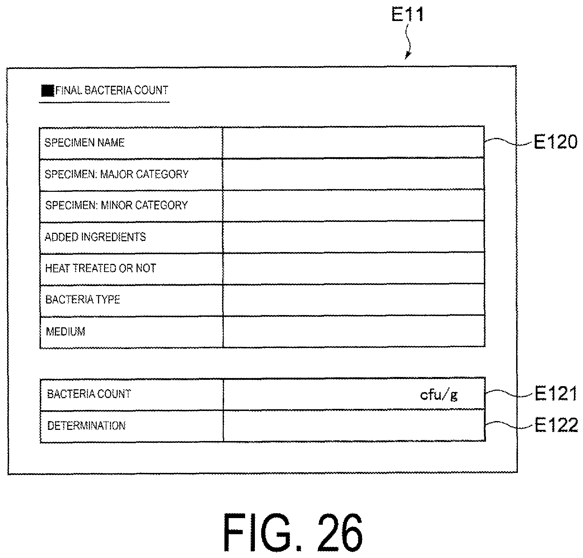

FIG. 26 illustrates a final bacteria count screen E11 that is yet another example of hygiene management information of other facility that is displayed. For example, the final bacteria count screen E11 includes a search condition E120, a bacteria count E121, and determination E122. In the search condition E120, at least any of the specimen name E81, the specimen: major category E82, the specimen: minor category E83, the added ingredients E84, the heat treated or not E85, the bacteria type E86, and the medium E87 input on the input screen E8 is displayed. In the bacteria count E121 and the determination E122, a bacteria count and determination results tagged with the items input on the input screen E8 are displayed. For example, the bacteria count E121 corresponds to the bacteria count D41 of the measurement data D4, and the determination E122 corresponds to the determination D44 of the measurement data D4.

As described above, at least any of the test contents screen E9, the image data E10, and the final bacteria count screen E11 is displayed, and thus a facility that is a member that can view data of other can view test contents, image data, and relationship (pass criteria) between a bacteria count and determination of other facility. Thus, to a facility that is a member that can view data of other, hygiene management information of other facility can be displayed, but information indicating the origin of the hygiene management information is not displayed. That is, the test contents, the image data, and the pass criteria of other facility are displayed, but of which facility these pieces of information are is not displayed. The information of the origin is not displayed in this way, and thus the privacy of a facility is ensured.

Next, effects of the hygiene management system 1, the hygiene management method, and the hygiene management program according to the present embodiment will be described. In the hygiene management system 1, the hygiene management method, and the hygiene management program according to an embodiment, the facility skill information reception unit 29 receives the facility information and the skill information of a facility and the facility skill information determining unit 30 determines a pass/fail for the facility information and the skill information. In the hygiene management system 1, the hygiene management method, and the hygiene management program according to the present embodiment, the authorization unit 32 authorizes a facility that is determined, as a result of a pass/fail determination for the facility information and the skill information, to have passed to view hygiene management information of other facility and/or to be viewable by other facility. Accordingly, the authorization is given to a facility determined to have passed as a result of a pass/fail determination for the facility information and the skill information, and thus, a facility that satisfies predetermined conditions can be allowed to share information with other facility. Thus, data can be shared while securing the reliability of database, and the usefulness of the hygiene management system can be improved.

The facility information may include ISO 17025 accreditation information. In this case, a facility can be determined based on ISO 17025 accreditation. Accordingly, a facility that performs testing, analysis, or measurement of a product with high accuracy can be determined to pass.

The skill information may include the level of external accuracy management. In this case, the level of external accuracy management with a purpose of ensuring the reliability of testing in a facility can be used to determine a pass/fail of a facility. Accordingly, since the pass/fail determination of a facility can be performed with higher accuracy, this contributes to high quality information of a database.

The exemplary hygiene management system 1 may include the facility skill information update unit 31 that requests a facility to update the facility information and the skill information in a certain amount of time after the authorization unit 32 has given the authorization. In this case, the facility skill information update unit 31 requests a facility to periodically update the facility information and the skill information. Accordingly, data of a database can be maintained at a high quality.

The hygiene management information extraction unit 34 may extract information of the number of test target substances obtained by testing (for example, the final bacteria count screen E11 including a bacteria count). In this case, since a facility can view the extracted number of test target substances, the facility can obtain information of the number of test target substances that other facility uses as a standard. Accordingly, on the basis of the obtained information of the number, validity confirmation of test results, setting of standards, and the like can be performed appropriately.

The hygiene management information extraction unit 34 may extract the image data E10 of a test target substance obtained by testing. In this case, since a facility can view the image data E10 of a test target substance, the facility can obtain information of the image data E10 of a test target substance of other facility. Thus, information of a test target substance of other facility can be visually understood as the image data E10.

The hygiene management information extraction unit 34 may extract test contents of a test target substance (for example, information displayed on the test contents screen E9). In this case, since test contents of other facility can be viewed, information relating to testing of a test target substance can further be shared.

The embodiments of the hygiene management system, the hygiene management method, and the hygiene management program according to the present disclosure are described above. However, the hygiene management system, the hygiene management method, and the hygiene management program according to the present disclosure are not limited to the embodiments described above, and modifications within the range of the scope defined by the claims and other applications may be made. That is, the configurations and the functions of the respective components of the hygiene management system, and the contents and the order of the respective steps of the hygiene management method can be modified as appropriate within the range of the scope defined by the claims. For instance, it will be appreciated that the hygiene management systems of this disclosure may also benefit food processing facilities need in making release determinations about food produced at the facilities or sub-facilities. Depending on location, local regulations, and other localized factors, facilities may be required to test for various types of target analytes, such as a broader all-count test for aerobic microbes at large, for targeted microbes (e.g., salmonella), and potentially to test according to different standards for the same targets in different jurisdictions. The data sharing techniques of this disclosure enables different facility managers to better determine, formulate, and fine-tune best practices for facility management, and to expedite decision making in this area. In the case of entities that might have, control, or operate multiple, even numerous, facilities, management may, if desired, leverage information from one facility to inform decisions for other facilities. In cases of a food production entity sourcing raw material from multiple suppliers, the food production entity can encourage suppliers to participate in the cross-facility information sharing scheme of this disclosure to obtain data about various suppliers to trace the source of problems and/or to institute quality uniformity across suppliers (e.g., in terms of supplier release determinations, etc.). In some instances, the systems of this disclosure may consolidate anonymized data from various facilities, whether the facilities are under related management or not. In these examples, the systems of this disclosure provide potential benefits to different companies that use common suppliers. Based on overlapping results from the shared hygiene management data, these companies can track a problem to individual supplier(s) using data such as source identifiers, types of testing employed at these sources, etc. By enabling cross-facility data sharing, the systems of this disclosure provide the potential advantages of improving consistency across facilities, by extracting hygiene management information having the same data structure and sometimes the same standards for multiple facilities, and disseminating this information among multiple facilities in a way that enables facility management to improve resource usage and attain or maintain standards compliance. The systems of this disclosure are scalable in that even facilities that are set up differently, conduct testing differently, or are disparate in location can make use of each other's data in a disaggregated way to suit the local management's needs. Because food safety testing varies from one food product to another (unlike in the case of many common clinical tests), the systems of this disclosure provide the advantage of disaggregated data when facility management may make the most use of it. By aggregating disparate data uploaded by multiple facilities into a central repository, and parsing out the by category (regardless of facility alignment) at the dissemination stage, the systems of this disclosure enable resource efficiency improvements as well as precision improvements across facilities to maintain widespread compliance while mitigating resource waste. The cross-facility information-sharing techniques of this disclosure are sufficiently scalable to accommodate a variety of testing protocols used at different facilities, such as direct tests or indirect tests (e.g., as might be the case with so-called "indicator testing"). While described mainly with respect to hygiene monitoring at food processing and packaging, it will be appreciated that the cross-facility information sharing techniques of this disclosure may also be applicable to pathogen testing or hygiene monitoring through a supply chain (e.g., for milking equipment, pasteurization equipment, bottling plants, etc. in an example of dairy food production), in addition to the horizontal data sharing aspects described herein with respect to achieving consistency across different last-mile customer-facing facilities. Similarly, the cross-facility information sharing techniques of this disclosure may also enable horizontal consistency across peer upstream processing facilities (e.g. across different bottling facilities in the dairy example above).

For example, in the embodiments described above, the example where the facility information includes ISO 17025 accreditation information and the skill information includes the level of external accuracy management is described. However, the facility information may include information other than ISO 17025 accreditation information, and the skill information may include information other than the level of external accuracy management.

The contents and the order of the respective steps can be modified as appropriate for the hygiene management program. Additionally, the processing procedure of the hygiene management method and the hygiene management program executed by at least one processor is not limited to the examples in the embodiments described above. For example, a portion of the above-described steps (processing) may be omitted, or the respective steps may be executed in a different order. Additionally, any two or more steps among the above-described steps may be combined, or a portion of the steps may be modified or deleted. Additionally, other step may be executed in addition to the above-described respective steps.

In the embodiments described above, the example where the hygiene management system 1 includes the server 20, and the server 20 includes the storage unit 21, the display control unit 22, the identification code outputting unit 23, the input unit 24, the pass/fail determining unit 25, the approval request unit 26, the test contents outputting unit 27, the falsification determining unit 28, the facility skill information reception unit 29, the facility skill information determining unit 30, the facility skill information update unit 31, the authorization unit 32, the keyword reception unit 33, the hygiene management information extraction unit 34, and the hygiene management information transmission unit 35 is described. However, any of the storage unit 21, the display control unit 22, the identification code outputting unit 23, the input unit 24, the pass/fail determining unit 25, the approval request unit 26, the test contents outputting unit 27, the falsification determining unit 28, the facility skill information reception unit 29, the facility skill information determining unit 30, the facility skill information update unit 31, the authorization unit 32, the keyword reception unit 33, the hygiene management information extraction unit 34, and the hygiene management information transmission unit 35 may be omitted.

In the embodiments described above, the plate F for detecting a bacteria count, the threshold value C6 indicating the number of bacteria that is a standard for determining a pass/fail for a bacteria count, the input unit 24 for inputting a bacteria count, and the pass/fail determining unit 25 for determining a pass/fail for a bacteria count are exemplified. However, instead of at least any of the plate F, the threshold value C6, the input unit 24, and the pass/fail determining unit 25, a plate for detecting a test target substance other than bacteria (for example, allergen), a threshold value indicating the number of a test target substance other than bacteria, an input unit for inputting the number of a test target substance other than bacteria, and a pass/fail determining unit for determining a pass/fail for a test target substance other than bacteria may be provided.

In the embodiments described above, the hygiene management system 1 that is a food hygiene management system for managing food, the hygiene management method, and the hygiene management program have been exemplified. However, an object managed by the hygiene management system, the hygiene management method, and the hygiene management program is not limited to food and can be modified as appropriate. For example, the hygiene management system, the hygiene management method, and the hygiene management program according to the present disclosure may be an environmental hygiene management system for managing environment.

### Reference Signs List

- 1: Hygiene management system
- 21: Storage unit
- 29: Facility skill information reception unit
- 30: Facility skill information determining unit
- 31: Facility skill information update unit
- 32: Authorization unit
- 33: Keyword reception unit
- 34: Hygiene management information extraction unit
- 35: Hygiene management information transmission unit
- D16: Facility information
- D17: Skill information
- D42: Image (image data)
- D44: Determination (determination of results of pass/fail for specimen)
- E10: Image data
- E122: Determination

## Claims

1. A hygiene management system (1) for use by a plurality of facilities performing testing of a test target substance, wherein the hygiene management system (1) is a client-server system and includes a server (20) capable of communicating with each of terminals (10) of the plurality of facilities, the server (20) comprising:
a storage unit (21) configured to store information received by the server (20) from the terminals (10) and information generated within the server (20) in a database;
a facility skill information reception unit (29) configured to receive facility information (D16) and skill information (D 17) of the facilities,
wherein the facility information (D16) includes ISO 17025 accreditation information for indicating whether a facility of the facilities is able to produce accurate test results, and wherein the skill information (D17) includes a level of external accuracy management for indicating a skill of a facility for testing;
a facility skill information determining unit (30) configured to determine a pass/fail for the facility information (D16) and the skill information (D17),
wherein a facility having acquired ISO 17025 accreditation information and having a level of external accuracy management equal to or greater than a value is determined to pass, and a facility not having acquired ISO 17025 accreditation information and having a level of external accuracy management less than the value is determined to fail, wherein the determination results (D44) by the facility skill information determining unit (30) are stored in the storage unit (21);
an authorization unit (32) configured to authorize a facility of the facilities determined to have passed by the facility skill information determining unit (30) to view hygiene management information of other facility of the facilities and/or be viewable by other facility of the facilities, wherein the hygiene management information is information relating to testing of the test target substance, and wherein the storage unit (21) is configured to store hygiene management information of the test target substance of the plurality of facilities;
a hygiene management information extraction unit (34) configured for extracting hygiene management information, which is stored in the storage unit (21), for an authorized facility; and
a hygiene management information transmission unit (35) configured for transmitting hygiene management information extracted by the hygiene management information extraction unit (34) to the terminal (10) of an authorized facility.

2. The hygiene management system (1) according to claim 1, comprising a facility skill information update unit (31) configured to request the facilities to update the facility information (D16) and the skill information (D17) in a certain amount of time after the authorization unit (32) has given the authorization.

3. The hygiene management system (1) according to claim 1 or 2, the server (20) further comprising:
a keyword reception unit (33) configured to receive, from the facilities, a search keyword for searching for the hygiene management information;
the hygiene management information extraction unit (34) further configured to extract, from the storage unit (21), hygiene management information matching the search keyword;
the hygiene management information transmission unit (35) further configured to transmit, to the facilities, the hygiene management information extracted by the hygiene management information extraction unit (34).

4. The hygiene management system (1) according to claim 3, wherein the hygiene management information extraction unit (34) extracts information of the number of the test target substances obtained by the testing.

5. The hygiene management system (1) according to claim 3 or 4, wherein the hygiene management information extraction unit (34) extracts image data (D42) of the test target substance obtained by the testing.

6. The hygiene management system (1) according to any one of claims 3 to 5, wherein the hygiene management information extraction unit (34) extracts test contents of the test target substance.

7. A hygiene management method for use by a plurality of facilities performing testing of a test target substance, whereby the method comprises the steps of:
receiving, from the facilities, facility information (D16) and skill information (D17) of the facilities,
wherein the facility information (D16) includes ISO 17025 accreditation information for indicating whether a facility of the facilities is able to produce accurate test results, and wherein the skill information (D17) includes a level of external accuracy management for indicating a skill of a facility for testing;
determining a pass/fail of the facilities from the facility information (D16) and the skill information (D17), wherein a facility having acquired ISO 17025 accreditation information and having a level of external accuracy management equal to or greater than a value is determined to pass, and a facility not having acquired ISO 17025 accreditation information and having a level of external accuracy management less than the value is determined to fail;
transmitting the pass/fail to the facilities; and
authorizing a facility of the facilities determined to have passed at the pass/fail determining step to view hygiene management information of other facility of the facilities and/or be viewable by other facility of the facilities, wherein the hygiene management information is information relating to testing of the test target substance;
storing the received facility information (D16) and skill information (D17) of the facilities, the determined pass/fail results (D44), and the hygiene management information of the test target substance of the plurality of facilities in a database;
extracting the hygiene management information, which is stored in the database, for an authorized facility; and
transmitting the extracted hygiene management information to an authorized facility.

8. A hygiene management program for use by a plurality of facilities performing testing of a test target substance, comprising instructions which, when the program is executed by a computer, cause the computer to carry out the steps of the method of claim 7.

## Patentansprüche

1. Hygienemanagementsystem (1) zur Verwendung durch eine Vielzahl von Einrichtungen, die Tests an einer zu testenden Zielsubstanz durchführen, wobei das Hygienemanagementsystem (1) ein Client-Server-System ist und einen Server (20) umfasst, der mit jedem der Endgeräte (10) der Vielzahl von Einrichtungen kommunizieren kann, wobei der Server (20) Folgendes umfasst:
eine Speichereinheit (21), die dafür konfiguriert ist, Informationen, die der Server (20) von den Endgeräten (10) empfängt, sowie Informationen, die innerhalb des Servers (20) generiert werden, in einer Datenbank speichert;
eine Einheit zum Empfang von Einrichtungs- und Kompetenzinformationen (29), die dafür konfiguriert ist, Einrichtungsinformationen (D16) und Kompetenzinformationen (D17) der Einrichtungen zu empfangen, wobei die Einrichtungsinformationen (D16) Akkreditierungsinformationen nach ISO 17025 enthalten, um anzugeben, ob eine der Einrichtungen in der Lage ist, genaue Testergebnisse zu liefern, und wobei die Kompetenzinformationen (D17) einen Grad des externen Genauigkeitsmanagements umfassen, der eine Qualifikation einer Einrichtung zum Testen angibt;
eine Einheit zum Bestimmen von Einrichtungs- und Kompetenzinformationen (30), die dafür konfiguriert ist, für die Einrichtungsinformationen (D16) und die Kompetenzinformationen (D17) ein Bestanden/Nicht-bestanden-Ergebnis zu bestimmen, wobei eine Einrichtung, die Akkreditierungsinformationen nach ISO 17025 erhalten hat und deren Grad des externen Genauigkeitsmanagements gleich oder größer als ein bestimmter Wert ist, als "bestanden" bestimmt wird, und eine Einrichtung, die keine Akkreditierungsinformationen nach ISO 17025 erhalten hat und deren Grad des externen Genauigkeitsmanagements unter dem Wert liegt, als "nicht bestanden" bestimmt wird, wobei die Bestimmungsergebnisse (D44) der Einheit zum Bestimmen von Einrichtungs- und Kompetenzinformationen (30) in der Speichereinheit (21) gespeichert werden;
eine Autorisierungseinheit (32), die dafür konfiguriert ist, eine der Einrichtungen, die von der Einheit zum Bestimmen von Einrichtungs- und Kompetenzinformationen (30) als "bestanden" bestimmt wurden, zum Einsehen von Informationen zum Hygienemanagement einer anderen der Einrichtungen zu autorisieren und/oder dazu, von einer der anderen Einrichtungen eingesehen werden zu können, wobei es sich bei den Informationen zum Hygienemanagement um Informationen handelt, die sich auf das Testen der zu testenden Zielsubstanz beziehen, und wobei die Speichereinheit (21) dafür konfiguriert ist, Informationen zum Hygienemanagement der zu testenden Zielsubstanz der Vielzahl von Einrichtungen zu speichern;
Einheit zur Extraktion von Informationen zum Hygienemanagement (34), die dafür konfiguriert ist, für eine autorisierte Einrichtung Informationen zum Hygienemanagement zu extrahieren, die in der Speichereinheit (21) gespeichert sind; und
Einheit zur Übertragung von Informationen zum Hygienemanagement (35), die dafür konfiguriert ist, die von der Einheit zur Extraktion von Informationen zum Hygienemanagement (34) extrahierten Informationen zum Hygienemanagement an das Endgerät (10) einer autorisierten Einrichtung zu übertragen.

2. Hygienemanagementsystem (1) nach Anspruch 1, umfassend eine Einheit (31) zur Aktualisierung von Einrichtungs- und Kompetenzinformationen, die dafür konfiguriert ist, die Einrichtungen aufzufordern, die Einrichtungsinformationen (D16) und die Kompetenzinformationen (D17) innerhalb eines bestimmten Zeitraums nach Erteilung der Autorisierung durch die Autorisierungseinheit (32) zu aktualisieren.

3. Hygienemanagementsystem (1) nach Anspruch 1 oder 2, wobei der Server (20) ferner umfasst:
eine Einheit zum Empfang von Schlüsselwörtern (33), die dafür konfiguriert ist, von den Einrichtungen einen Suchbegriff zum Suchen nach den Informationen zum Hygienemanagement zu empfangen;
wobei die Einheit zur Extraktion von Informationen zum Hygienemanagement (34) ferner dafür konfiguriert ist, aus der Speichereinheit (21) Informationen zum Hygienemanagement zu extrahieren, die mit dem Suchbegriff übereinstimmen;
wobei die Einheit zur Übertragung von Informationen zum Hygienemanagement (35) ferner dafür konfiguriert ist, die von der Einheit zur Extraktion von Informationen zum Hygienemanagement (34) extrahierten Informationen zum Hygienemanagement an die Einrichtungen zu übertragen.

4. Hygienemanagementsystem (1) nach Anspruch 3, wobei die Einheit zur Extraktion von Informationen zum Hygienemanagement (34) Informationen über die Anzahl der beim Testen ermittelten zu testenden Zielsubstanzen extrahiert.

5. Hygienemanagementsystem (1) nach Anspruch 3 oder 4, wobei die Einheit zur Extraktion von Informationen zum Hygienemanagement (34) Bilddaten (D42) der beim Testen ermittelten zu testenden Zielsubstanzen extrahiert.

6. Hygienemanagementsystem (1) nach einem der Ansprüche 3 bis 5, wobei die Einheit zur Extraktion von Informationen zum Hygienemanagement (34) Testinhalte der zu testenden Zielsubstanzen extrahiert.

7. Verfahren zum Hygienemanagement zur Verwendung durch eine Vielzahl von Einrichtungen, die Tests an einer zu testenden Zielsubstanz durchführen, wobei das Verfahren die folgenden Schritte umfasst:
Empfangen von Einrichtungsinformationen (D16) und Kompetenzinformationen (D17) über die Einrichtungen von den Einrichtungen, wobei die Einrichtungsinformationen (D16) Akkreditierungsinformationen nach ISO 17025 enthalten, um anzugeben, ob eine der Einrichtungen in der Lage ist, genaue Testergebnisse zu liefern, und wobei die Kompetenzinformationen (D17) einen Grad des externen Genauigkeitsmanagements umfassen, der eine Qualifikation einer Einrichtung zum Testen angibt;
Bestimmen eines Bestanden/Nicht-bestanden-Ergebnisses der Einrichtung anhand der Einrichtungsinformationen (D16) und der Kompetenzinformationen (D17), wobei eine Einrichtung, die Akkreditierungsinformationen nach ISO 17025 erhalten hat und deren Grad des externen Genauigkeitsmanagements gleich oder größer als ein bestimmter Wert ist, als "bestanden" bestimmt wird, und eine Einrichtung, die keine Akkreditierungsinformationen nach ISO 17025 erhalten hat und deren Grad des externen Genauigkeitsmanagements unter dem Wert liegt, als "nicht bestanden" bestimmt wird;
Übertragen des Bestanden/Nicht-bestanden-Ergebnisses an die Einrichtungen; und
Autorisieren einer der Einrichtungen, für die im Schritt zur Bestimmung von "Bestanden" oder "Nicht bestanden" ein Bestanden-Ergebnis bestimmt wurde, zum Einsehen von Informationen zum Hygienemanagement einer anderen Einrichtung der IO-Einrichtungen und/oder dazu, von einer der anderen Einrichtungen eingesehen werden zu können, wobei es sich bei den Informationen zum Hygienemanagement um Informationen handelt, die sich auf das Testen der zu testenden Zielsubstanz beziehen;
Speichern der empfangenen Einrichtungsinformationen (D16) und Kompetenzinformationen (D17) der Einrichtungen, der bestimmten Bestanden/Nicht-bestanden-Ergebnisse (D44) sowie der Informationen zum Hygienemanagement der zu testenden Zielsubstanz der Vielzahl von Einrichtungen in einer Datenbank;
Extrahieren der in der Datenbank gespeicherten Informationen zum Hygienemanagement für eine autorisierte Einrichtung; und
Übertragen der abgerufenen Informationen zum Hygienemanagement an eine autorisierte Einrichtung.

8. Hygienemanagementprogramm zur Verwendung durch eine Vielzahl von Einrichtungen, die das Testen einer zu testenden Zielsubstanz durchführen, umfassend Anweisungen, die, wenn das Programm von einem Computer ausgeführt wird, den Computer veranlassen, die Schritte des Verfahrens nach Anspruch 7 auszuführen.

## Revendications

1. Système de gestion de l'hygiène (1) destiné à être utilisé par une pluralité d'installations réalisant l'essai d'une substance cible d'essai, dans lequel le système de gestion d'hygiène (1) est un système client-serveur et inclut un serveur (20) capable de communication avec chacun des terminaux (10) de la pluralité d'installations, le serveur (20) comprenant :
une unité de stockage (21) configurée pour stocker des informations reçues par le serveur (20) des terminaux (10) et des informations générées au sein du serveur (20) dans une base de données ;
une unité de réception d'informations de compétence d'installation (29) configurée pour recevoir des informations d'installation (D16) et des informations de compétence (D17) des installations, dans lequel les informations d'installation (D16) incluent des informations d'accréditation ISO 17025 destinées à indiquer si une installation des installations est capable de produire des résultats d'essai exacts, et dans lequel les informations de compétence (D17) incluent un niveau de gestion d'exactitude externe destiné à indiquer une compétence d'une installation destinée à des essais ;
une unité de détermination d'informations de compétence d'installation (30) configurée pour déterminer une conformité/non conformité pour les informations d'installation (D16) et les informations de compétence (D17), dans lequel une installation ayant acquis des informations d'accréditation ISO 17025 et ayant un niveau de gestion d'exactitude externe supérieur ou égal à une valeur est déterminée comme étant conforme, et une installation n'ayant pas acquis des informations d'accréditation ISO 17025 et ayant un niveau de gestion d'exactitude externe inférieur à la valeur est déterminée comme n'étant pas conforme, dans lequel les résultats de détermination (D44) par l'unité de détermination d'informations de compétence d'installation (30) sont stockés dans l'unité de stockage (21) ;
une unité d'autorisation (32) configurée pour autoriser une installation des installations déterminées comme étant conformes par l'unité de détermination d'informations de compétence d'installation (30) à visionner des informations de gestion d'hygiène d'une autre installation des installations et/ou à être visionnée par une autre installation des installations, dans lequel les informations de gestion d'hygiène sont des informations concernant l'essai de la substance cible d'essai, et dans lequel l'unité de stockage (21) est configurée pour stocker des informations de gestion d'hygiène de la substance cible d'essai de la pluralité d'installations ;
une unité d'extraction d'informations de gestion d'hygiène (34) configurée pour extraire des informations de gestion d'hygiène, qui sont stockées dans l'unité de stockage (21), pour une installation autorisée ; et
une unité de transmission d'informations de gestion d'hygiène (35) configurée pour transmettre des informations de gestion d'hygiène extraites par l'unité d'extraction d'informations de gestion d'hygiène (34) au terminal (10) d'une installation autorisée.

2. Système de gestion d'hygiène (1) selon la revendication 1, comprenant une unité de mise à jour d'informations de compétence d'installation (31) configurée pour demander aux installations de mettre à jour les informations d'installation (D16) et les informations de compétence (D17) dans une certaine quantité de temps une fois que l'unité d'autorisation (32) a donné l'autorisation.

3. Système de gestion d'hygiène (1) selon la revendication 1 ou 2, le serveur (20) comprenant en outre :
une unité de réception de mot-clé (33) configurée pour recevoir, à partir des installations, un mot-clé de recherche destiné à rechercher les informations de gestion d'hygiène ;
l'unité d'extraction d'informations de gestion d'hygiène (34) configurée en outre pour extraire, à partir de l'unité de stockage (21), des informations de gestion d'hygiène correspondant au mot-clé de recherche ;
l'unité de transmission d'informations de gestion d'hygiène (35) configurée en outre pour transmettre, aux installations, les informations de gestion d'hygiène extraites par l'unité d'extraction d'informations de gestion d'hygiène (34).

4. Système de gestion d'hygiène (1) selon la revendication 3, dans lequel l'unité d'extraction d'informations de gestion d'hygiène (34) extrait des informations du nombre des substances cibles d'essai obtenues par l'essai.

5. Système de gestion d'hygiène (1) selon la revendication 3 ou 4, dans lequel l'unité d'extraction d'informations de gestion d'hygiène (34) extrait des données d'image (D42) de la substance cible d'essai obtenue par l'essai.

6. Système de gestion d'hygiène (1) selon l'une quelconque des revendications 3 à 5, dans lequel l'unité d'extraction d'informations de gestion d'hygiène (34) extrait des contenus d'essai de la substance cible d'essai.

7. Procédé de gestion d'hygiène destiné à être utilisé par une pluralité d'installations réalisant l'essai d'une substance cible d'essai, par lequel le procédé comprend les étapes suivantes :
la réception, à partir des installations, d'informations d'installation (D16) et d'informations de compétence (D17) des installations, dans lequel les informations d'installation (D16) incluent des informations d'accréditation ISO 17025 destinées à indiquer si une installation des installations est capable de produire des résultats d'essai exacts, et dans lequel les informations de compétence (D17) incluent un niveau de gestion d'exactitude externe destiné à indiquer une compétence d'une installation destinée à des essais ;
la détermination d'une conformité/non conformité des installations à partir des informations d'installation (D16) et des informations de compétence (D17), dans lequel une installation ayant acquis des informations d'accréditation ISO 17025 et ayant un niveau de gestion d'exactitude externe supérieur ou égal à une valeur est déterminée comme étant conforme, et une installation n'ayant pas acquis d'informations d'accréditation ISP 17025 et ayant un niveau de gestion d'exactitude externe inférieur à la valeur est déterminée comme étant non conforme ;
la transmission de la conformité/non conformité aux installations ; et
l'autorisation d'une installation des installations déterminée comme étant conforme lors de l'étape de détermination de conformité/non conformité à visionner des informations de gestion d'hygiène d'une autre installation des installations et/ou à être visionnée par une autre installation des installations, dans lequel les informations de gestion d'hygiène sont des informations concernant l'essai de la substance cible d'essai ;
le stockage des informations d'installation (D16) et des informations de compétences (D17) reçues des installations, des résultats de conformité/non conformité déterminés (D44), et des informations de gestion d'hygiène de la substance cible d'essai de la pluralité d'installations dans une base de données ;
l'extraction des informations de gestion d'hygiène, qui sont stockées dans la base de données, pour une installation autorisée ; et
la transmission des informations de gestion d'hygiène extraites à une installation autorisée.

8. Programme de gestion d'hygiène destiné à être utilisé par une pluralité d'installations réalisant l'essai d'une substance cible d'essai, comprenant des instructions qui, lorsque le programme est exécuté par un ordinateur, amène l'ordinateur à effectuer les étapes du procédé selon la revendication 7.
